(19) Europäisches Patentamt
European Patent Office
Office européen des brevets

(11) Numéro de publication: 0 385 848
A1

(12) **DEMANDE DE BREVET EUROPEEN**

(21) Numéro de dépôt: 90400535.2

(51) Int. Cl.[5]: C07D 263/58, A61K 31/42

(22) Date de dépôt: 27.02.90

(30) Priorité: 28.02.89 FR 8902554

(43) Date de publication de la demande: 05.09.90 Bulletin 90/36

(84) Etats contractants désignés: AT BE CH DE DK ES FR GB GR IT LI LU NL SE

(71) Demandeur: ADIR ET COMPAGNIE
22, rue Garnier
F-92201 Neuilly sur Seine(FR)

(72) Inventeur: Lesieur, Daniel
20, rue de Verdun
F-59147 Gondecourt(FR)
Inventeur: Lespagnol, Charles
115, Avenue Pasteur
F-59130 Lambersart(FR)
Inventeur: Bonnet, Jacqueline
19, rue Charcot
F-75013 Paris(FR)

(54) **Nouveaux dérivés benzoxazolinoniques, leurs procédés de préparation et les compositions pharmaceutiques qui les contiennent.**

(57) Composés de formule générale (I) :

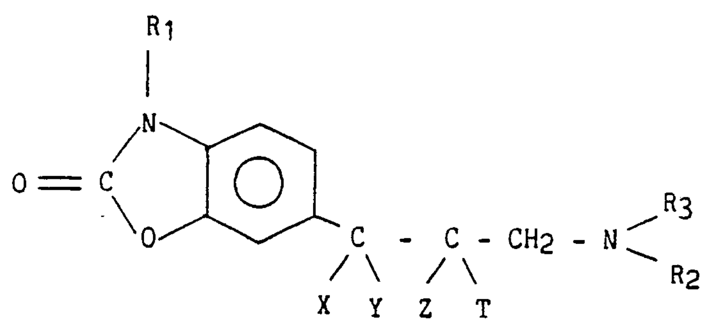

(I)

dans laquelle :
$R_1$ représente un atome d'hydrogène ou alkyle inférieur éventuellement substitué par un groupement hydroxy,
$R_2$ et $R_3$ :
a- identiques ou différents représentent un atome d'hydrogène, un alkyle inférieur, un alkényle inférieur, un aryle substitué ou non, un alkyle inférieur aryle substitué ou non,
b- sont tels qu'ils constituent avec l'azote qui les porte un système hétérocyclique, mono ou bicyclique, saturé ou non, contenant au maximum 3 hétéroatomes par cycle, substitué ou non, à l'exception des systèmes aryl - 1 pipéraziniques, X représente un atome d'hydrogène,
Y représente un atome d'hydrogène ou un hydroxyle,
ou bien X et Y représentent un atome d'oxygène à la condition que $R_1$ soit alors différent du groupement méthyle,
Z représente un atome d'hydrogène ou forme avec Y une liaison $\pi$,
T représente un atome d'hydrogène ou un alkyle inférieur,
leurs énantiomères, épimères, diastéréoisomères, leurs sels d'ammonium quaternaires, ainsi que leurs sels d'addition à acide pharmaceutiquement acceptable.

EP 0 385 848 A1

Médicaments.

# NOUVEAUX DERIVES BENZOXAZOLINIQUES, LEURS PROCEDES DE PREPARATION ET LES COMPOSITIONS PHARMACEUTIQUES QUI LES CONTIENNENT

La présente invention concerne de nouveaux dérivés de la benzoxazolinone, leurs préparations et les compositions pharmaceutiques qui les contiennent.

De nombreux dérivés de la benzoxazolinone ont été décrits en thérapeutique comme possédant des activités pharmacologiques très variées. Le brevet FR 73.23280 décrit des acyl - 6 benzoxazolinones en tant qu'analgésiques. Le brevet FR 80.20861 décrit notamment des (amino - 2 éthyl) - 6 benzoxazolinones et (amino acétyl) - 6 benzoxazolinones utilisables dans le traitement de l'hypertension artérielle ainsi que dans celui des syndromes douloureux. Le brevet FR 82.19812 décrit des (amino -2 éthyl) - 6 benzoxazolinones utilisables en thérapeutique dans le traitement des troubles du sommeil et des troubles du caractère et du comportement.

La demanderesse a maintenant découvert des dérivés benzoxazolinoniques doués d'une activité analgésique dépourvue d'activité anti-inflammatoire d'un niveau nettement plus intéressant que celui des dérivés décrits dans les brevets FR 73.23280 et FR 80.20861. Les composés de la présente invention sont en effet doués d'une activité analgésique pure de haut niveau. Or, la plupart des substances analgésiques non morphiniques connues à ce jour possèdent également une activité anti-inflammatoire (exemples : salicylés, pyrazolés...) et ils interviennent par conséquent sur les processus intervenant dans l'inflammation. Ceux-ci impliquent de très nombreux médiateurs chimiques (prostaglandines, thomboxane A2...) ; il s'ensuit donc de multiples effets secondaires dont les plus connus sont : attaque de la muqueuse gastrique avec possibilité d'ulcères. Outre les dérangements qu'ils occasionnent, ces effets parallèles interdisent l'usage de ces produits chez de nombreux sujets qui y sont particulièrement sensibles. En étant dépourvus de toute activité anti-inflammatoire, les composés de la présente invention n'interviennent pas sur les médiateurs de l'inflammation et sont donc dépourvus des effets secondaires mentionnés précédemment. Cette caractéristique, jointe pour quelques uns d'entre eux à leur absence totale de toxicité et à leur haut niveau d'activité rend certains composés de la présente invention utilisables en tant qu'analgésiques d'une façon beaucoup plus sûre et sans les restrictions d'usage habituellement connues pour la grande majorité de ces produits. Certains dérivés de l'invention présentent en outre d'intéressantes activités sur la tension artérielle et sur le système nerveux central.

Plus spécifiquement, l'invention concerne les dérivés de formule générale (I) :

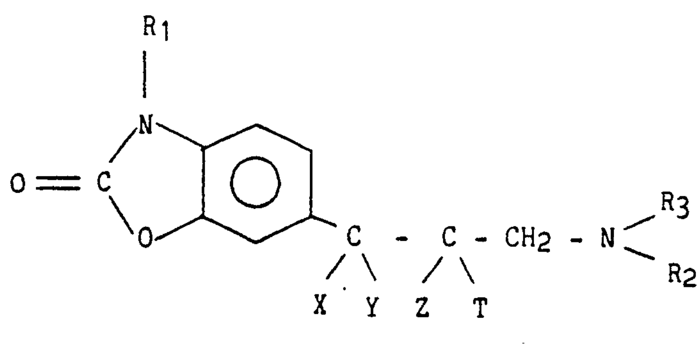

(I)

dans laquelle :

$R_1$ représente un atome d'hydrogène ou un groupement alkyle inférieur éventuellement substitué par un groupement hydroxyle,

$R_2$ et $R_3$ identiques ou différents représentent :

- un atome d'hydrogène,
- un groupement alkyle inférieur linéaire ou ramifié,
- un groupement alkényle inférieur linéaire ou ramifié,
- un groupement aryle ou alkyle inférieur aryle dans lesquels la partie aryle est éventuellement substituée par un ou plusieurs atomes d'halogène, ou groupements alkyle inférieurs éventuellement substitués par un ou plusieurs atomes d'halogène, ou groupements alkoxy inférieurs,

ou bien, $R_2$ et $R_3$ constituent avec l'atome d'azote auquel ils sont liés un système hétérocyclique, mono ou bicyclique, saturé ou non contenant un, deux ou trois hétéroatomes par cycle choisis parmi azote, oxygène ou soufre, substitué ou non par un halogène, un groupement alkyle inférieur, alkoxy inférieur ou aryle éventuellement substitué par un ou plusieurs atomes d'halogène, à la condition que $R_2$ et $R_3$ ne constituent

pas avec l'atome d'azote auquel ils sont liés un système aryl - 1 pipérazinique,
X représente un atome d'hydrogène,
Y représente un atome d'hydrogène ou un groupement hydroxyle,
ou bien, X et Y représentent ensemble un atome d'oxygène à la condition que dans ce cas $R_1$ soit différent du groupement méthyle,
Z représente un atome d'hydrogène ou bien Z forme avec Y une liaison $\pi$,
T représente un atome d'hydrogène ou un groupement alkyle inférieur,
le terme inférieur indiquant que les groupements ainsi qualifiés comptent de 1 à 6 atomes de carbone,
leurs énantiomères, diastéréoisomères et épimères, leurs sels d'ammonium quaternaire ainsi que leurs sels d'addition à un acide pharmaceutiquement acceptable.

Parmi les acides que l'on peut utiliser pour salifier les composés de formule générale (I), on peut citer, à titre non limitatif les acides chlorhydrique, sulfurique, phosphorique, tartrique, malique, maléique, fumarique, oxalique, méthane-sulfonique, éthane-sulfonique, camphorique, citrique etc...

L'invention s'étend aussi à deux procédés d'obtention des composés de formule (I).

Selon les composés de l'invention que l'on souhaite obtenir, il pourra en effet être avantageux d'utiliser soit l'un, soit l'autre procédé.

Le premier procédé de préparation des composés de formule (I), particulièrement intéressant pour l'obtention des composés de formule (I) dans lesquels X, Y, Z représentent chacun un atome d'hydrogène, peut toutefois être appliqué pour les dérivés où X, Y, Z ont d'autres valeurs, utilise comme matière première un dérivé de formule (II) :

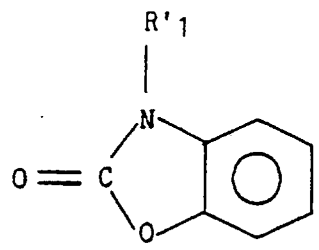

(II)

dans laquelle, $R'_1$ représente un atome d'hydrogène ou un groupement alkyle inférieur,
obtenu, par exemple, par réaction de l'orthoaminophénol sur l'urée suivie, lorsque $R'_1$ est différent de H, d'une alkylation à l'azote,
que l'on soumet à l'action d'un chlorure d'acide de formule (III) :

$Cl - CO - \underset{T}{HC} - CH_2 - A$ (III)

dans laquelle, T a la même signification que dans la formule (I), A représentant un atome d'halogène,
ou bien de l'anhydride d'acide correspondant,
en présence de chlorure d'aluminium dans le diméthyl formamide selon les conditions de THYES et Coll. (J. Med. Chem. 1983, 26, 6, 800 - 807),
pour obtenir un dérivé de formule (IV) :

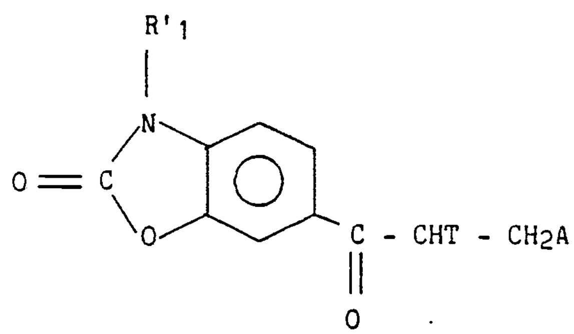

(IV)

dans laquelle $R'_1$, T et A ont la même signification que précédemment,
qui, si on le désire, est soumis à réduction par un trialkylsilane en milieu acide selon les conditions décrites par WEST et Coll. (J. Org. Chem. 1973, 38, (15), 2675 - 2681), pour conduire à un dérivé de formule (V) :

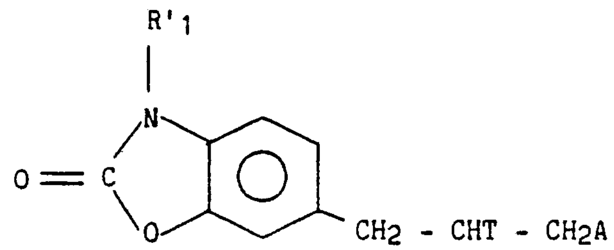

(V)

dans laquelle $R'_1$, T et A ont la même signification que précédemment,
le dérivé de formule (IV), ou le dérivé de formule (V), selon la formule du dérivé de formule (I) que l'on souhaite obtenir, étant alors soumis à l'action d'une amine de formule (VI) :

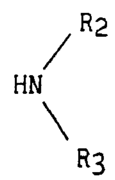

(VI)

dans laquelle $R_2$ et $R_3$ ont la même signification que dans la formule (I),
dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi, en présence d'un excès de l'amine choisie ou d'un capteur de l'hydracide formé tel que la triéthylamine,
pour conduire à un dérivé de formule (I/A) :

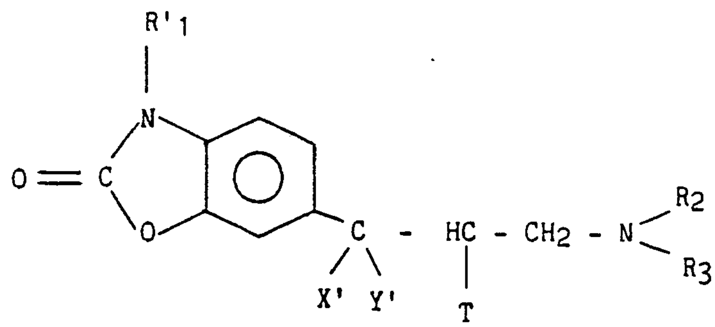

(I/A)

dans laquelle, selon que la matière première utilisée est un dérivé de formule (IV) ou (V),
$X'$ et $Y'$ représentent ensemble un atome d'oxygène, ou bien X et Y représentent chacun simultanément un atome d'hydrogène,
$R'_1$, $R_2$, $R_3$ et T ayant la même signification que précédemment,
qui, si on le désire, est salifié par un acide pharmaceutiquement acceptable ou qui peut, lorsque $X'$ et $Y'$ représentent ensemble un atome d'oxygène, si on le désire, être soumis,
* ou bien, à un agent d'hydrogénation choisi parmi un hydrure mixte de métal alcalin, comme par exemple le borohydrure de sodium, ou un cyanohydrure mixte de métal alcalin, comme le cyano borohydrure de sodium, préférentiellement en milieu d'alcool aliphatique inférieur, pour conduire à un dérivé de formule (I/B) - majoritairement sous la configuration thréo lorsque T ne représente pas un atome d'hydrogène - :

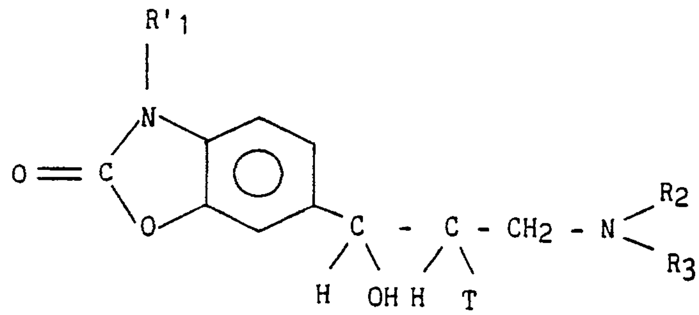

(I/B)

cas particulier des dérivés de formule (I), dans laquelle :
$R'_1$, $R_2$, $R_3$ et T ont la même signification que précédemment,
X représente un atome d'hydrogène,
Y un groupement hydroxyle et Z un atome d'hydrogène,
dont on sépare, si on le désire, les isomères et/ou que l'on salifie par un acide pharmaceutiquement acceptable,
* ou bien à hydrogénation catalytique, sous chauffage et pression dans un solvant choisi parmi alcool aliphatique inférieur ou dioxanne, pour conduire à un dérivé de formule (I/B) - essentiellement sous la configuration érythro lorsque T ne représente pas un atome d'hydrogène - dont on sépare, si on le désire, les isomères et que l'on salifie le cas échéant par un acide pharmaceutiquement acceptable,
dérivé de formule (I/B) qui, quelque soit le procédé selon lequel il a été obtenu peut être, si on le désire, traité par un agent déshydratant,
préférentiellement choisi parmi les hydracides, pour conduire à un dérivé de formule (I/C) :

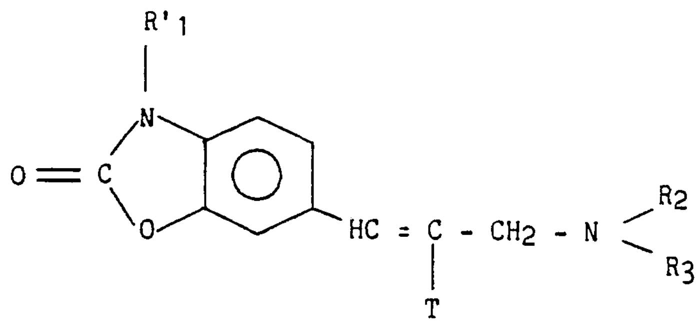

(I/C)

majoritairement sous forme d'isomère trans, cas particulier de dérivés de formule (I), dans laquelle :
$R'_1$, $R_2$, $R_3$ et T ont la même signification que précédemment
X représente un atome d'hydrogène,
Z forme avec Y une liaison $\pi$,
dont, on sépare, si on le désire, les isomères cis-trans par une technique usuelle telle que la chromatographie sur colonne de silice ou la cristallisation,
et qui, si on le souhaite, peut être salifié par un acide pharmaceutiquement acceptable, dérivé de formule (I/A), (I/B) ou (I/C),
qui, lorsque $R'_1$ représente un atome d'hydrogène, peut être traité en présence d'une base forte par un dérivé de formule $X-(CH_2)n-OH$,
dans laquelle X représente un atome d'halogène et n est compris entre 1 et 6 pour conduire à un dérivé de formule (I) pour lequel $R_1$ représente un groupement alkyle inférieur substitué par un groupement hydroxyle,
dérivé de formule (I) que l'on peut traiter si on le désire par un agent d'alkylation classique tel que le sulfate de méthyle pour conduire à un sel d'ammonium quaternaire.

Le second procédé d'obtention des dérivés de la présente invention est inapplicable pour les dérivés pour lesquels R représente un atome d'hydrogène ou un groupement alkyle substitué par un groupement hydroxyle.

Ce second procédé se caractérise en ce que l'on acyle un dérivé de formule (II) obtenu comme indiqué précédemment :

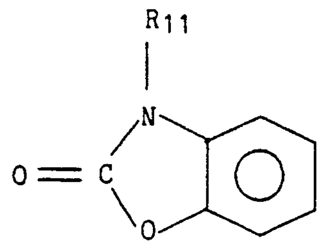

(II)

dans laquelle $R_{11}$ représente un groupement alkyle inférieur par un acide de formule (VII) :

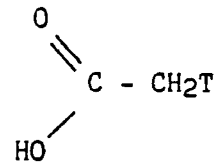

(VII)

dans laquelle T a la même signification que dans la formule (I) ou le chlorure, ou l'anhydride de l'acide correspondant selon les conditions décrites dans le brevet FR 73.23280,
pour obtenir un dérivé de formule (VIII) :

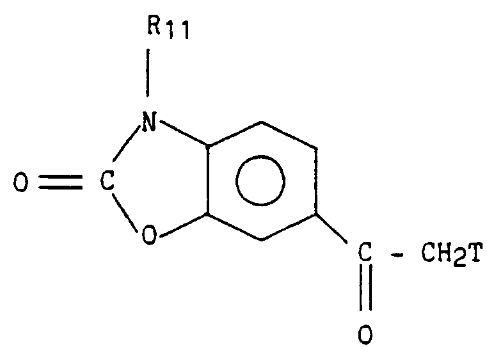

(VIII)

dans laquelle, $R_{11}$ représente un groupement alkyle inférieur, et T a la même signification que dans la formule (I),
que l'on traite ensuite,
* ou bien, selon les conditions de la réaction de Mannich, bien connues de l'homme de l'Art, en présence de trioxyméthylène et de l'amine de formule (VI) :

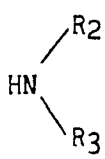

(VI)

dans laquelle, $R_2$ et $R_3$ ont la même signification que dans la formule (I), pour obtenir un dérivé de formule (I/A1) :

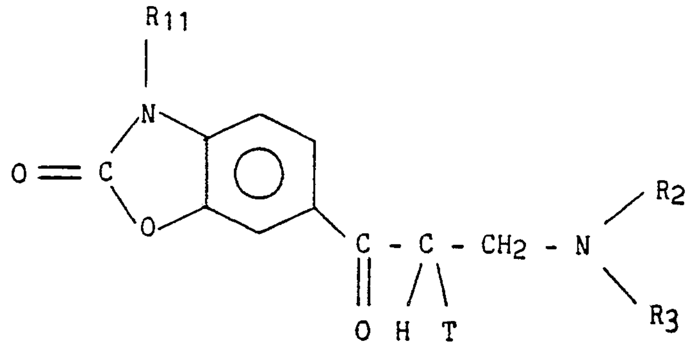

(I/A1)

cas particulier des dérivés de formules (I/A) et (I), dans laquelle :
$R_{11}$ représente un groupement alkyle inférieur, et $R_2$, $R_3$, T ont la même signification que dans la formule (I),
X et Y représentent ici simultanément un atome d'oxygène et Z un atome d'hydrogène,
* ou bien par le bisdiméthylaminométhane en présence d'anhydride acétique pour obtenir un produit de formule générale (IX) :

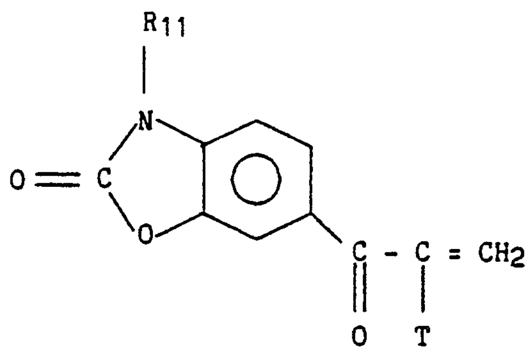

(IX)

dans laquelle :
T a la même signification que dans la formule (I) et $R_{11}$ représente un groupement alkyle inférieur,
que l'on traite par une amine de formule (VI), dans un solvant polaire à une température comprise entre la température ambiante et la température d'ébullition du milieu réactionnel,
pour conduire à un dérivé de formule (I/A1) précédemment défini,
qui, lorsque T ne représente pas un atome d'hydrogêne peut être, si on le désire, séparé en ses isomères, et que l'on salifie, si on le désire par un acide pharmaceutiquement acceptable et qui,
si on le souhaite, peut être soumis :
* ou bien préférentiellement en milieu d'alcool aliphatique inférieur à un agent d'hydrogénation préférentiellement un hydrure mixte de métal alcalin ou un cyanohydrure mixte de métal alcalin comme par exemple le borohydrure de sodium, ou le cyanoborohydrure de sodium,
pour conduire à un dérivé de formule (I/B) majoritairement sous la configuration thréo (lorsque T ne représente pas un atome d'hydrogène) :

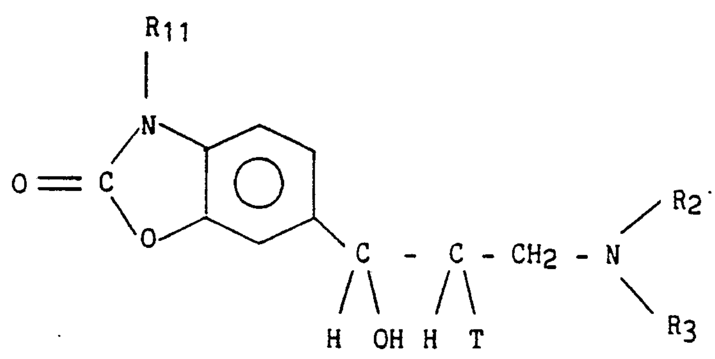

(I/B)

cas particulier des dérivés de formule (I), dans laquelle :
$R_{11}$, $R_2$, $R_3$ et T ont la même signification que précédemment,
X représente un atome d'hydrogène,
Y un groupement hydroxyle et Z un atome d'hydrogène,
dont on sépare, si on le désire, les isomères et que l'on peut salifier par un acide pharmaceutiquement acceptable,
* ou bien à hydrogénation catalytique dans un solvant choisi parmi alcool aliphatique inférieur ou dioxanne pour conduire à un dérivé de formule (I/B) essentiellement sous la configuration érythro -lorsque T ne représente pas un atome d'hydrogène - dont on sépare, si on le désire, les isomères et que l'on salifie le cas échéant par un acide pharmaceutiquement acceptable,
dérivé de formule (I/B) que, le cas échéant, on soumet à un agent déshydratant choisi préférentiellement parmi les hydracides pour conduire à un dérivé de formule (I/C), majoritairement sous forme d'isomères trans :

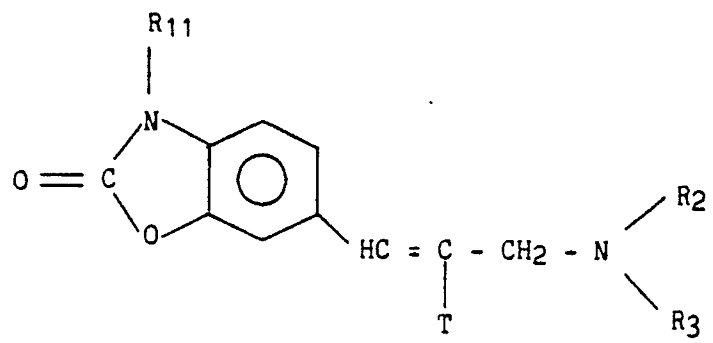

(I/C)

cas particulier de dérivés de formule (I), dans laquelle :
$R_{11}$, $R_2$, $R_3$ et T ont la même signification que dans les dérivés de formule (I),
X représente un atome d'hydrogène,
Z forme avec Y une liaison $\pi$,
dont, si on le désire, on sépare les isomères cis-trans par une technique usuelle telle que la chromatographie sur colonne de silice ou la cristallisation,
et que l'on salifie, si on le désire, par un acide pharmaceutiquement acceptable,
qui, si on le désire, est soumis à une réaction d'hydrogénation catalytique préférentiellement à température et pression ambiantes, et en présence de Nickel de Raney en milieu d'alcool aliphatique inférieur ou de dioxanne pour obtenir un dérivé de formule (I/D) :

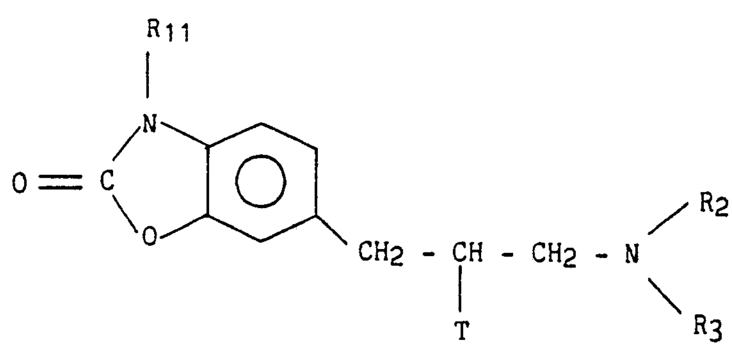

(I/D)

dans laquelle :
$R_{11}$, $R_2$, $R_3$ et T ont la même signification que précédemment,
X, Y et Z représente chacun simultanément un atome d'hydrogène.
dont on sépare le cas échéant les isomères lorsque T ne représente pas un atome d'hydrogène,
et, que l'on salifie éventuellement par un acide pharmaceutiquement acceptable ou que l'on transforme en sel d'ammonium quaternaire par action d'un agent d'alkylation comme indiqué précédemment.

Les dérivés de formule (I/D) peuvent également être obtenus à partir des dérivés de formule (IX) :

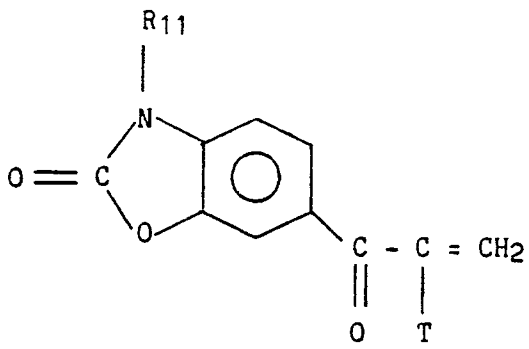

(IX)

dans laquelle :
$R_{11}$ et T ont la même signification que précédemment,
que l'on traite par un hydracide pour obtenir un dérivé de formule (IV) :

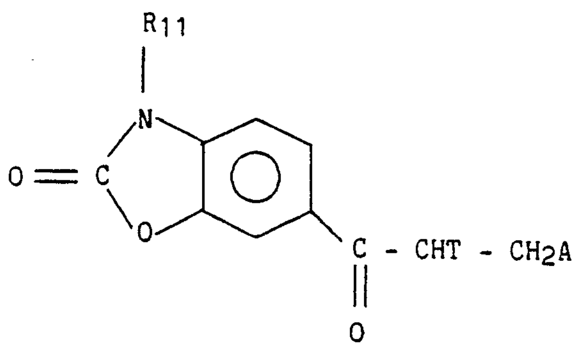

(IV)

dans laquelle $R_{11}$ et T ont la même signification que précédemment et A la même signification que dans la formule (III),
dont on sépare, si on le désire, les isomères lorsque T ne représente pas un atome d'hydrogène,
que l'on soumet à réduction par un trialkylsilane en milieu acide selon les conditions décrites par WEST et Coll. (J. Org. Chem. 1973, 38, (15), 2675 - 2681), pour conduire à un dérivé de formule (V) :

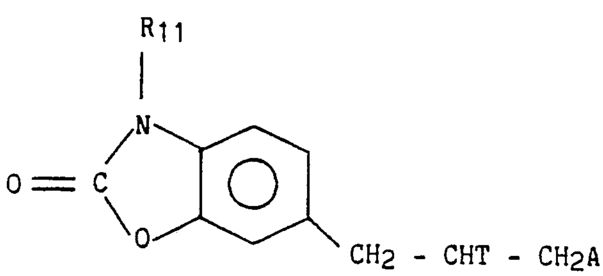

(V)

dans laquelle $R_{11}$ et T ont la même signification que précédemment, et A la même signification que dans la formule (III),
que l'on soumet à l'action d'une amine de formule (VI) :

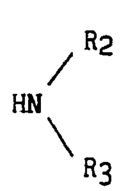

(VI)

dans laquelle $R_2$ et $R_3$ ont la même signification que dans la formule (I), dans un solvant préférentiellement

choisi parmi acétone, acétonitrile, acétate d'éthyle, alcool aliphatique inférieur, dioxanne, benzène, toluène, à une température comprise entre la température ambiante et la température d'ébullition du solvant choisi en présence d'un excès de l'amine choisie ou d'un capteur de l'hydracide formé tel que la triéthylamine, pour conduire à un dérivé de formule (I/D) ci-dessus référencé, que l'on salifie si on le désire par un acide pharmaceutiquement acceptable ou que l'on transforme en un sel d'ammonium quaternaire apr un agent d'alkylation.

Les composés de formule (I) possèdent des propriétés pharmacologiques intéressantes.

En particulier ces dérivés ont révélé une activité analgésique intéressante ainsi que pour certains d'entre eux une activité sur le système nerveux central et sur la tension artérielle.

L'étude pharmacologique des dérivés de l'invention a en effet montré qu'ils étaient peu toxiques, doués d'une activité analgésique pure et donc dépourvus d'inconvénients inhérents à la plupart des composés non morphiniques présentant cette activité (action ulcérigène sur les muqueuses...). Ce spectre d'activité rend donc les composés de la présente invention intéressants dans un certain nombre d'indications telles que algies rhumatismales, névralgies, lombo-sciatiques, névralgies cervico-brachiales, algies traumatiques telles que entorses, fractures, luxations, douleurs post-traumatiques, douleurs post-opératoires, douleurs dentaires, douleurs neurologiques telles que névralgies faciales, douleurs viscérales telles que coliques néphrétiques, dysménorrhées, chirurgie proctologique, douleurs de la sphère O.R.L., pancréatites, algies diverses, céphalées, douleurs des cancéreux...

La présente invention a également pour objet les compositions pharmaceutiques contenant les produits de formule (I), seuls ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 centigramme et 4 grammes par 24 heures.

Les exemples suivants illustrent l'invention et ne la limitent en aucune façon.

Les spectres infrarouges ont été réalisés à partir d'une pastille de bromure de potassium contenant approximativement 1 % du produit à analyser.

**EXEMPLE 1 : METHYL-3 [(PIPERIDINYL-1)-3 PROPYL)]-6 BENZOXAZOLINONE (CHLORHYDRATE)**

**STADE A : (CHLORO-3 PROPIONYL)-6 BENZOXAZOLINONE**

Dans une fiole rodée contenant 37,4 g (0,28 mole) de chlorure d'aluminium anhydre, introduire goutte à goutte et sous agitation 6,02 ml (0,078 mole) de diméthylformamide.

Munir la fiole d'un réfrigérant à reflux et porter dans un bain d'huile à une température voisine de 40-45° C. Introduire 0,04 mole de méthyl - 3 benzoxazolinone et 0,44 mole de chlorure d'acide chloro - 3 propionique. Chauffer à une température voisine de 75° C pendant 2 heures 30 minutes. Après refroidissement, verser le mélange réactionnel dans 300 g de glace, acidifier par l'acide chlorhydrique concentré et agiter pendant 1 heure 30 minutes. Essorer le précipité obtenu, laver à l'eau et sécher. Recristalliser dans l'éthanol.

Rendement : 88 %

Point de fusion : 187 ° C

Infrarouge : $\nu$ CO (carbamate) : 1765 $cm^{-1}$

$\nu$ CO (cétone) : 1660 $cm^{-1}$

**STADE B : METHYL-3 (CHLORO-3 PROPYL)-6 BENZOXAZOLINONE**

Dans une fiole rodée, dissoudre 0,02 mole de méthyl - 3 (chloro - 3 propionyl) - 6 benzoxazolinone obtenu au stade précédent, dans 22,8 g (0,2 mole) d'acide trifluoroacétique. Ajouter goutte à goutte et en refroidissant 0,044 mole de triéthylsilane. Adapter une garde à chlorure de calcium et poursuivre l'agitation pendant 72 heures. Verser alors le milieu réactionnel dans de l'eau glacée, essorer le précipité obtenu,

sécher et recristalliser dans l'hexane.
Rendement : 80 %
Point de fusion : 70 °C
Infrarouge : ν CO : 1775 $cm^{-1}$

**STADE C : METHYL-3 [(PIPERIDINYL-1)-3 PROPYL]-6 BENZOXAZOLINONE (CHLORHYDRATE)**

Dans une fiole rodée de 250 $cm^3$ munie d'un réfrigérant, dissoudre 0,02 mole de méthyl - 3 (chloro - 3 propyl) - 6 benzoxazolinone obtenu au stade B, dans de l'acétonitrile. Ajouter sous agitation magnétique 0,04 mole de pipéridine et porter à reflux pendant 48 heures. Après refroidissement de la solution, filtrer l'insoluble formé puis évaporer le filtrat au bain-marie sous vide. Reprendre le résidu par 500 $cm^3$ d'eau, alcaliniser par une solution aqueuse de soude à 10 %. Essorer le précipité, laver à l'eau jusqu'à neutralité de la phase aqueuse puis dissoudre le composé dans l'hexane et faire barboter un courant d'acide chlorhydrique gazeux. Essorer, sécher, recristalliser dans de l'éthanol.
Rendement : 69 %
Point de fusion : 238 °C
Infrarouge : ν CO : 1770 $cm^{-1}$

**EXEMPLE 2 : METHYL-3 [(METHYL-4 PIPERAZINYL-1)-3 PROPYL]-6 BENZOXAZOLINONE (DICHLORHYDRATE)**

Dans une fiole rodée de 250 $cm^3$ munie d'un réfrigérant, dissoudre 0,02 mole de méthyl - 3 (chloro - 3 propyl) - 6 benzoxazolinone obtenue au stade B, de l'exemple 1 dans du dioxanne. Ajouter sous agitation 0,04 mole de N - méthyl pipérazine et porter à reflux pendant 48 heures. Après refroidissement de la solution filtrer l'insoluble, puis évaporer le filtrat au bain-marie sous vide. Reprendre le résidu par 500 $cm^3$ d'eau, alcaliniser par une solution aqueuse de bicarbonate de sodium à 10 % et extraire par du chloroforme. Sécher la phase organique sur du chlorure de calcium, filtrer et évaporer à sec. On obtient un produit huileux repris par de l'acétone. Après barbotage d'acide chlorhydrique gazeux dans cette solution, essorer, sécher et recristalliser dans un mélange méthanol/acétone.
Rendement : 53 %
Point de fusion : > 270 °C
Infrarouge : νCO : 1770 $cm^{-1}$

**EXEMPLE 3 : METHYL-3[(PYRROLIDINYL-1)-3 PROPYL]-6 BENZOXAZOLINONE (CHLORHYDRATE)**

Dans une fiole rodée de 250 $cm^3$ munie d'un réfrigérant, dissoudre 0,02 mole de méthyl - 3 (chloro - 3 propyl) - 6 benzoxazolinone obtenue au stade B de l'exemple 1 dans de l'acétonitrile. Ajouter sous agitation 0,04 mole de pyrrolidine, 0,02 mole d'iodure de potassium et 0,02 mole de carbonate de sodium et porter à reflux pendant 4 jours. Après refroidissement de la solution, filtrer l'insoluble, puis évaporer le filtrat au bain-marie sous vide. Reprendre le résidu par de l'éther, filtrer l'insoluble restant et faire barboter un courant d'acide chlorhydrique gazeux dans cette solution. Essorer, sécher et recristalliser dans l'acétone.
Rendement : 69 %
Point de fusion : 174 °C
Infrarouge : νCO : 1770 $cm^{-1}$

**EXEMPLE 4 : METHYL-3 (HYDROXY-1 MORPHOLINO-3 PROPYL)-6 BENZOXAZOLINONE**

**STADE A : METHYL-3 (MORPHOLINO-3 PROPIONYL)-6 BENZOXAZOLINONE**

Dans une fiole rodée de 250 $cm^3$ munie d'un réfrigérant, dissoudre 0,03 mole d'acétyl - 6 méthyl - 3 benzoxazolinone obtenue comme décrit dans le brevet Fr. 73 23 280 et 0,045 mole de chlorhydrate de morpholine dans 150 $cm^3$ d'éthanol absolu. Ajouter 0,045 mole de trioxyméthylène et acidifier par l'acide chlorhydrique. Porter à reflux pendant 64 heures. Essorer le précipité formé, laver à l'acétone, placer en

suspension dans l'eau, alcaliniser par la soude. Extraire à plusieurs reprises au chloroforme, sécher les phases organiques sur chlorure de sodium, filtrer et évaporer au bain-marie sous vide et recristalliser dans l'éthanol.
Rendement : 55 %
Point de fusion : 134 °C
Infrarouge : ν CO (carbamate) : 1770 $cm^{-1}$
ν CO (Cétone) : 1660 $cm^{-1}$

**STADE B : METHYL-3 (HYDROXY-1 MORPHOLINO-3 PROPYL)-6 BENZOXAZOLINONE**

Dans une fiole de 250 $cm^3$ munie d'une agitation magnétique, dissoudre 0,01 mole de méthyl - 3 (morpholino - 3 propionyl) - 6 benzoxazolinone préparé au stade A dans 200 $cm^3$ de méthanol. Ajouter très lentement et sous agitation 0,02 mole de borohydrure de sodium. Maintenir l'agitation 4 heures à température ambiante. Evaporer le milieu réactionnel au bain-marie sous vide. Reprendre le résidu par l'eau et extraire plusieurs fois au chloroforme. Filtrer, évaporer à sec au bain-maire sous vide et recristalliser dans le cyclohexane.
Rendement : 68 %
Point de fusion : 115 °C
Infrarouge : ν CO : 1755 $cm^{-1}$

**EXEMPLE 5 : METHYL-3 [(MORPHOLINO-3) PROPENYL-1]-6 BENZOXAZOLINONE**

Dans une fiole de 250 $cm^3$ dissoudre 0,015 mole de méthyl - 3 (hydroxy - 1 morpholino - 3 propyl) - 6 benzoxazolinone obtenue dans l'exemple 4 dans 50 $cm^3$ d'acide bromhydrique à 47 % et agiter la solution à la température ambiante pendant 2 heures. Essorer le précipité obtenu, le laver à l'acétone, le placer en suspension dans l'eau alcaliniser par la soude. Extraire à plusieurs reprises au chloroforme, réunir et sécher les phases organiques sur chlorure de calcium, filtrer, évaporer à sec sous vide et recristalliser dans du propanol. Rendement : 64 %
Point de fusion : 132 °C
Infrarouge : ν CO : 1765 $cm^{-1}$

**EXEMPLE 6 : METHYL-3 (MORPHOLINO-3 PROPYL)-6 BENZOXAZOLINONE**

Dans une fiole conique de 500 $cm^3$ munie d'un robinet 3 voies et d'une agitation magnétique, dissoudre 0,01 mole de méthyl - 3 [morpholino - 3 propenyl - 1] - 6 benzoxazolinone obtenue dans l'exemple 5 dans du méthanol puis ajouter 0,5 g de nickel de Raney. Agiter sous atmosphère d'hydrogène à température et pression ordinaires. Après absorption de la quantité théorique d'hydrogène, filter le milieu réactionnel, évaporer à sec le filtrat au bain-marie sous vide. Reprendre le résidu par de l'eau, acidifier par l'acide chlorhydrique, essorer le précipité obtenu et recristalliser dans de l'acétate d'éthyle.
Rendement : 86 %
Point de fusion : 226 °C
Infrarouge : ν CO : 1770 $cm^{-1}$

**EXEMPLE 7 : METHYL-3 (MORPHOLINO-3 PROPYL)-6 BENZOXAZOLINONE**

En procédant comme dans l'exemple 1, mais en remplaçant la pipéridine (stade C) par la morpholine on obtient le produit attendu.

**EXEMPLE 8 : METHYL-3 (DIETHYLAMINO-3 PROPYL)-6 BENZOXAZOLINONE**

En procédant comme dans l'exemple 1, mais en remplaçant la pipéridine (stade C) par la N,N diéthylamine, on obtient le produit attendu.
Point de fusion : 131-132 °C

**EXEMPLE 9 : (MORPHOLINO-3 PROPYL)-6 BENZOXAZOLINONE (CHLORHYDRATE)**

**STADE A : (CHLORO-3 PROPIONYL)-6 BENZOXAZOLINONE**

Dans une fiole rodée, peser 56,1 g (0,42 mole) de chlorure d'aluminium puis ajouter à l'aide d'une ampoule à brome 10 $cm^3$ de diméthylformamide et laisser sous agitation jusqu'à ce que le mélange devienne homogène. Ajouter alors 8,1 g (0,06 mole) de benzoxazolinone et laisser sous agitation 10 minutes avant d'ajouter 7 $cm^3$ (0,072 mole) de chlorure d'acide chloro - 3 propionique. Maintenir ce mélange dans un bain d'huile à 80-85° C pendant environ 2 heures et 30 minutes. Après refroidissement, verser le mélange réactionnel sur de l'eau glacée. Essorer le précipité et le laver à l'eau jusqu'à neutralité. Recristalliser dans de l'éthanol.
Rendement : 70 %
Point de fusion : 166° C
Infrarouge : $\nu$ CO (carbamate) : 1760 $cm^{-1}$
$\nu$ CO (cétone) : 1655 $cm^{-1}$

**STADE B : (CHLORO-3 PROPYL)-6 BENZOXAZOLINONE**

Dans une fiole rodée introduire 9 g (0,04 mole) de (chloro - 3 propionyl) - 6 benzoxazolinone obtenue au stade A et 31 $cm^3$ d'acide trifluoroacétique. Ajouter goutte à goutte à l'aide d'une ampoule à brome et sous agitation 13,5 $cm^3$ (0,09 mole) de triéthylsilane. Remplacer l'ampoule à brome par une garde de chlorure de calcium et laisser sous agitation à température ambiante pendant 48 heures. Verser alors le mélange réactionnel sur de l'eau glacée et laisser sous agitation pendant 2 heures. Essorer le précipité et le laver à l'eau jusqu'à neutralité. Recristalliser dans du toluène.
Rendement : 70 %
Point de fusion : 127° C
Infrarouge : $\nu$CO : 1770 $cm^{-1}$

**STADE C : (MORPHOLINO-3 PROPYL)-6 BENZOXAZOLINONE (CHLORHYDRATE)**

Dans un ballon dissoudre 4,2 g (0,02 mole) de (chloro - 3 propyl) -6 benzoxazolinone dans 100 $cm^3$ d'acétonitrile. Ajouter 2,0 g (0,02 mole) de morpholine et 2,4 g (0,02 mole) de triéthylamine et laisser sous agitation à reflux pendant 5 jours. Après refroidissement, évaporer à sec le mélange réactionnel, reprendre plusieurs fois par de l'acétone anhydre et filtrer le chlorhydrate de triéthylamine formé. Evaporer l'acétone, reprendre par un mélange acétone/hexane et faire barboter un courant d'acide chlorhydrique gazeux dans cette solution. Essorer et sécher le précipité puis recristalliser dans l'acétone.
Rendement : 43 %
Point de fusion : 120° C
Infrarouge : $\nu$CO : 1750 $cm^{-1}$

**EXEMPLE 10 : [(PIPERIDINYL-1)-3 PROPYL]-6 BENZOXAZOLINONE**

En procédant comme dans l'exemple 9, mais en remplaçant la morpholine (stade C) par la pipéridine, on obtient le produit attendu.

**EXEMPLE 11 : [(METHYL-4 PIPERAZINYL-1)-3 PROPYL]-6 BENZOXAZOLINONE**

En procédant comme dans l'exemple 9, mais en remplaçant la morpholine (stade C) par la méthyl - 4 pipérazine, on obtient le produit attendu.

**EXEMPLE 12 : [(PYRROLIDINYL-1 )-3 PROPYL]-6 BENZOXAZOLINONE**

En procédant comme dans l'exemple 9, mais en remplaçant la morpholine (stade C) par la pyrrolidine, on obtient le produit attendu.

**EXEMPLE 13 : (DIETHYLAMINO-3 PROPYL)-6 BENZOXAZOLINONE**

En procédant comme dans l'exemple 9, mais en remplaçant la morpholine (stade C) par la diéthylamine, on obtient le produit attendu.

**EXEMPLE 14 : METHYL-3 ((THIAZOLIDINYL-3) 3 PROPYL]-6 BENZOXAZOLINONE**

En procédant comme dans l'exemple 1, mais en remplaçant la pipéridine (stade C) par la thiazolidine, on obtient le produit attendu.

**EXEMPLE 15 : [(THIAZOLIDINYL-3)-3 PROPYL]-6 BENZOXAZOLINONE**

En procédant comme dans l'exemple 9, mais en remplaçant la morpholine (stade C) par la thiazolidine, on obtient le produit attendu.

**EXEMPLE 16 : METHYL-3 (DIMETHYLAMINO-3 PROPYL)-6 BENZOXAZOLINONE**

En procédant comme dans l'exemple 1, mais en remplaçant la pipéridine (stade C) par la diméthylamine, on obtient le produit attendu.

**EXEMPLE 17 : (DIMETHYLAMINO-3 PROPYL)-6 BENZOXAZOLINONE**

En procédant comme dans l'exemple 9, mais en remplaçant la morpholine (stade C) par la diméthylamine, on obtient le produit attendu.

**EXEMPLE 18 : METHYL-3 [(INDOLINYL-1)-3 PROPYL-6 BENZOXAZOLINONE**

En procédant comme dans l'exemple 1, mais en remplaçant la pipéridine (stade C) par l'indoline, on obtient le produit attendu.

**EXEMPLE 19 : (INDOLINYL-1)-3 PROPYL-6 BENZOXAZOLINONE**

En procédant comme dans l'exemple 9, mais en remplaçant la morpholine (stade C) par l'indoline, on obtient le produit attendu.

**EXEMPLE 20 : METHYL-3 [HYDROXY-1 (PIPERIDINYL-1)-3 PROPYL]-6 BENZOXAZOLINONE**

En procédant comme dans l'exemple 4, mais en remplaçant le chlorhydrate de morpholine (stade A) par le chlorhydrate de pipéridine, on obtient le produit attendu.

**EXEMPLE 21 : METHYL-3 [(PIPERIDINYL-3 PROPENYL-1]-6 BENZOXAZOLINONE**

En procédant comme dans l'exemple 5, mais en remplaçant la méthyl -3 (hydroxy - 1 morpholino - 3 propyl) - 6 benzoxazolinone par la méthyl -3 (hydroxy - 1 pipéridinyl - 3 propyl) - 6 benzoxazolinone (exemple 20), on obtient le produit attendu.

**EXEMPLE 22 : METHYL-3 [(PIPERIDINYL-1)-3 PROPYL)-6 BENZOXAZOLINONE**

En procédant comme dans l'exemple 6, mais en remplaçant la méthyl -3 (morpholino - 3 propenyl - 1) - 6 benzoxazolinone par la méthyl - 3 (pipéridinyl - 3 propenyl - 1) - 6 benzoxazolinone (exemple 21), on obtient le produit attendu.

**EXEMPLE 23 : METHYL-3 [HYDROXY-1(METHYL-4 PIPERAZINYL-1)-3 PROPYL]-6 BENZOXAZOLINONE**

En procédant comme dans l'exemple 4, mais en remplaçant le chlorhydrate de morpholine (stade A) par le dichlorhydrate de méthyl - 1 pipérazine, on obtient le produit attendu.

**EXEMPLE 24 : METHYL-3 [METHYL-4 PIPERAZINYL-1)-3 PROPENYL-1]-6 BENZOXAZOLINONE**

En procédant comme dans l'exemple 5, mais en remplaçant la méthyl -3 [hydroxy - 1 morpholino - 3 propyl] - 6 benzoxazolinone par la méthyl -3 [hydroxy - 1 (méthyl - 4 pipérazinyl - 1) - 3 propyl] - 6 benzoxazolinone (exemple 23), on obtient le produit attendu.

**EXEMPLE 25 : METHYL-3 [(METHYL-4 PIPERAZINYL-1)-3 PROPYL]-6 BENZOXAZOLINONE**

En procédant comme dans l'exemple 6, mais en remplaçant la méthyl -3 (morpholino - 3 propenyl - 1) - 6 benzoxazolinone par la méthyl - 3 [(méthyl - 4 pipérazinyl - 1) - 3 propenyl - 1] - 6 benzoxazolinone (exemple 24), on obtient le produit attendu.

**EXEMPLE 26 : METHYL-3 (HYDROXY-1 DIMETHYLAMINO-3 PROPYL)-6 BENZOXAZOLINONE**

En procédant comme dans l'exemple 4, mais en remplaçant le chlorhydrate de morpholine (stade A) par le chlorydrate de diméthylamine, on obtient le produit attendu.

**EXEMPLE 27 : METHYL-3 (DIMETHYLAMINO-3 PROPENYL-1)-6 BENZOXAZOLINONE**

En procédant comme dans l'exemple 5, mais en remplaçant la méthyl -3 (hydroxy - 1 morpholino - 3 propyl) - 6 benzoxazolinone par la méthyl -3 (hydroxy - 1 diméthylamino - 3 propyl) - 6 benzoxazolinone (exemple 26), on obtient le produit attendu.

**EXEMPLE 28 : METHYL-3 (DIMETHYLAMINO-3 PROPYL)-6 BENZOXAZOLINONE**

En procédant comme dans l'exemple 6, mais en remplaçant la méthyl -3 (morpholino - 3 propenyl - 1) - 6 benzoxazolinone par la méthyl - 3 (diméthylamino - 3 propenyl - 1) - 6 benzoxazolinone (exemple 27), on obtient le produit attendu.

**EXEMPLE 29 : METHYL-3 [HYDROXY-1 (PYRROLIDINYL-1)-3 PROPYL]-6 BENZOXAZOLINONE**

En procédant comme dans l'exemple 4, mais en remplaçant le chlorhydrate de morpholine (stade A) par le dichlorhydrate de pyrrolidine, on obtient le produit attendu.

**EXEMPLE 30 : METHYL-3 [(PYRROLIDINYL-1)-3 PROPENYL-1]-6 BENZOXAZOLINONE**

En procédant comme dans l'exemple 5, mais en remplaçant la méthyl -3 (hydroxy - 1 morpholino - 3 propyl) - 6 benzoxazolinone par la méthyl -3 [hydroxy - 1 (pyrrolidinyl - 1) - 3 propyl] - 6 benzoxazolinone (exemple 29), on obtient le produit attendu.

**EXEMPLE 31 : METHYL-3 [(PYRROLIDINYL-1)-3 PROPYL]-6 BENZOXAZOLINONE**

En procédant comme dans l'exemple 6, mais en remplaçant la méthyl -3 (morpholino - 3 propenyl - 1) - 6 benzoxazolinone par la méthyl - 3 [(pyrrolidinyl - 1) - 3 propenyl - 1] - 6 benzoxazolinone (exemple 30), on obtient le produit attendu.

**EXEMPLE 32 : METHYL-3 [(HYDROXY-1 (THIAZOLIDINYL-3)-3 PROPYL]-6 BENZOXAZOLINONE**

En procédant comme dans l'exemple 4, mais en remplaçant le chlorhydrate de morpholine (stade A) par le chlorhydrate de thiazolidine, on obtient le produit attendu.

**EXEMPLE 33 : METHYL-3 [(THIAZOLIDINYL-3)-3 PROPENYL-1]-6 BENZOXAZOLINONE**

En procédant comme dans l'exemple 5, mais en remplaçant la méthyl -3 (hydroxy - 1 morpholino - 3 propyl) - 6 benzoxazolinone par la méthyl -3 [hydroxy - 1 (thiazolidinyl - 3) - 3 propyl] - 6 benzoxazolinone (exemple 32), on obtient le produit attendu.

**EXEMPLE 34 : METHYL-3 [(THIAZOLIDINYL-3)-3 PROPYL-1]-6 BENZOXAZOLINONE**

En procédant comme dans l'exemple 6, mais en remplaçant la méthyl -3 (morpholino - 3 propényl - 1) - 6 benzoxazolinone par la méthyl - 3 [(thiazolidinyl - 3) - 3 propényl - 1] - 6 benzoxazolinone (exemple 33), on obtient le produit attendu.

**EXEMPLE 35 : (MORPHOLINO-3 PROPYL)-6 (HYDROXY-2 ETHYL)-3 BENZOXAZOLINONE (CHLORHYDRATE)**

Ajouter 0,01 mole du chlorhydrate de (morpholino-3 propyl)-6 benzoxazolinone obtenu dans l'exemple 9, dans de l'éthylate de sodium. Laisser en contact une heure, évaporer à sec, reprendre par 20 $cm^3$ de D.M.F. et ajouter à froid sous agitation 1,2 équivalent de bromo-2 éthanol. Agiter toute une nuit à température ambiante. Evaporer à sec le D.M.F., reprendre par l'eau et extraire la phase organique par le chloroforme. Sécher sur CaCl2, filtrer et évaporer à sec.
Reprendre par un mélange acétone/éther, filtrer et faire barboter un courant d'acide chlorhydrique gazeux ; le produit précipite. Recristalliser dans le propanol.
Rendement : 50 %
Point de fusion : 209-201 ° C

**EXEMPLE 36 : (N METHYLMORPHOLINO-3 PROPYL)-6 METHYL-3 BENZOXAZOLINONE, METHANE SULFONATE**

Dissoudre 0,03 mole de méthyl-3 (chloro-3 propyl)-6 benzoxazolinone dans 100 $cm^3$ d'acétonitrile. Ajouter 5,2 $cm^3$ de morpholine et laisser à reflux sous agitation pendant 48 heures. Refroidir, essorer, évaporer le filtrat, reprendre par l'eau, alcaliniser par de la soude à 19%. Extraire la phase organique au chloroforme, sécher sur chlorure de calcium, filtrer et évaporer à sec.
Reprendre par le chloroforme anhydre et ajouter 0,03 mole de sulfate de méthyle. Laisser à reflux sous agitation pendant une heure. Le produit précipite ; essorer et recristalliser dans l'éthanol.
Rendement : 65%
Point de fusion : 142° C

**ETUDE PHARMACOLOGIQUE DES DERIVES DE L'INVENTION**

**EXEMPLE 37 : ETUDE DE LA TOXICITE AIGUE**

La toxicité aiguë a été appréciée après administration orale à des lots de 8 souris (26±2 grammes) d'une dose de 1000 $mg.kg^{-1}$. Les animaux ont été observés à intervalles réguliers au cours de la première journée et quotidiennement pendant les 2 semaines suivant le traitement.

Il apparaît que les composés de l'invention sont totalement atoxiques. Aucun décès n'est observé après administration d'une dose de 1000 $mg.kg^{-1}$. On ne constate pas de troubles après administration de cette dose.

**EXEMPLE 38 : ETUDE DE L'ACTIVITE ANALGESIQUE**

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 119, 1874, 1954). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02 %. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une $ED_{50}$, dose entraînant une activité de 50%, a été déterminée pour chaque produit.

Il est apparu que certains composés de l'invention possèdent une activité analgésique très intéressante. Ainsi, l'$ED_{50}$ du composé de l'exemple 6 est voisine de 5 $mg.kg^{-1}$.

A titre de comparaison l'administration d'une dose de 100 $mg.kg^{-1}$ des dérivés du brevet Fr 73.23280 provoquait un pourcentage d'analgésie -dans un test comparable - de l'ordre de 25 à 60 % et le composé du brevet Fr 80.20861 dont l'activité analgésique est la plus intéressante avait dans ce même test de Siegmund une $ED_{50}$ de 9 $mg.kg^{-1}$ soit près de 2 fois supérieure à celle du produit le plus intéressant de la présente invention.

**EXEMPLE 39 : ETUDE DE L'ACTIVITE ANTI-INFLAMMATOIRE**

Le potentiel anti-inflammatoire des composés à été recherché sur un modèle d'inflammation aiguë provoquée par injection sous-cutanée d'une solution de carragénine au niveau de la patte postérieure de rat, selon une technique inspirée de la méthode de WINTERCH ; E.A. RISLEY, G.N. NUSS - (Proc. Soc. Exp. Med. 111, 554, 1962). Les rats (100-120 g), randomisés en lots de 8, ont été traités (y compris les témoins qui reçoivent l'excipient) 1 heure avant l'injection locale d'une suspension à 0,5% de carragénine (type IV, Sigma ; 0,1 ml de rat). L'oedème est déterminé 3 heures après l'injection, par mesure pléthysmométrique (pléthysmomètre à eau UGO BASILE) du volume de chacune des pattes postérieures (oedème = volume de la patte enflammée diminué du volume de la patte non enflammée).

Il apparaît que les produits de l'invention n'ont aucune activité sur ce test. En comparaison, les produits du brevet Fr 73.23280 possèdent une activité anti-inflammatoire.

**EXEMPLE 40 : ANTAGONISME DE LA RESERPINE**

L'antagonisme de la réserpine a été apprécié après administration de réserpine 2,5 $mg.kg^{-1}$) à un lot de 6 souris. Quatre heures après, le composé à tester est administré par voire intrapéritonéale. Un lot témoin ne reçoit pas de produit.

Deux paramètres sont observés : la température rectale et le ptosis. Chez les témoins l'administration de réserpine entraîne la fermeture de l'oeil et une baisse importante de la température rectale.

L'administration de certains composés de l'invention antagonise les effets de la réserpine, ce qui montre l'activité antidépressive de ces dérivés.

**EXEMPLE 41 : ACTIVITE ANTIHYPERTENSIVE**

La pression artérielle a été déterminée à la queue du rat selon la méthode décrite apr BYROM et

WILSON(1938). Cette méthode consiste à mesurer la pression nécessaire pour interrompre le flux sanguin de l'artère candale. Dans ce but, un brassard pneumatique en caoutchouc relié à un électrosphygmomanomètre NARCO type PE 300 est fixé au niveau de la queue des rats à 2 centimètres de la base de manière à comprimer l'artère candale.

Un capteur de pulsation type pneumatique permet d'ausculter l'artère à 1 cm en aval du brassard. La valeur de la pression artérielle systolique est celle pour laquelle on observe, au cours du dégonflage du manchon, la réapparition des oscillations systolo-diastoliques.

Les produits de l'invention sont administrés par voie orale en suspension dans du sirop de gomme sous un volume de 1 ml.kg-1.

La pression est mesurée avant tout traitement, 2 heures et 24 heures après le traitement.

Certains produits de l'invention abaissent la pression artérielle de façon significative.

**EXEMPLE 42 : COMPOSITION PHARMACEUTIQUE : COMPRIME**

Comprimés dosés à 20 mg de méthyl-3 (morpholino-3 propyl)-6 benzoxazolinone.

| Formule de préparation pour 1000 comprimés. | |
|---|---|
| Méthyl-3 (morpholino-3 propyl)-6 benzoxazolinone ..... | 20 g |
| Amidon de blé ..... | 15 g |
| Amidon de maïs ..... | 15 g |
| Lactose ..... | 65 g |
| Stéarate de magnésium ..... | 2 g |
| Silice ..... | 1 g |
| Hydroxypropylcellulose ..... | 2 g |

**Revendications**

1/ Composés de formule générale (I) :

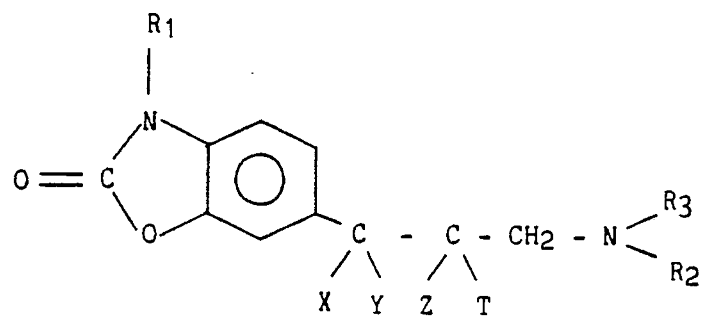

(I)

dans laquelle :
$R_1$ représente un atome d'hydrogène ou groupement alkyle inférieur éventuellement substitué par un groupement hydroxyle,
$R_2$ et $R_3$ identiques ou différents représentent :
- un atome d'hydrogène,
- un groupement alkyle inférieur linéaire ou ramifié,
- un groupement alkényle inférieur linéaire ou ramifié,
- un groupement aryle ou alkyle inférieur aryle dans lesquels la partie aryle est éventuellement substituée par un ou plusieurs atomes d'halogène ou groupements alkyle inférieurs éventuellement substitués par un ou plusieurs atomes d'halogène ou groupements alkoxy inférieurs,
ou bien, $R_2$ et $R_3$ constituent avec l'atome d'azote auquel ils sont liés un système hétérocyclique, mono ou bicyclique, saturé ou non contenant un, deux ou trois hétéroatomes par cycle choisis parmi azote, oxygène

ou soufre, substitué ou non par un halogène, un groupement alkyle inférieur, alkoxy inférieur ou aryle éventuellement substitué par un ou plusieurs atomes d'halogène, à la condition que $R_2$ et $R_3$ ne constituent pas avec l'atome auquel ils sont attachés un système aryl - 1 pipérazénique,
X représente un atome d'hydrogène,
Y représente un atome d'hydrogène ou un groupement hydroxyle,
ou bien X et Y représentent ensemble un atome d'oxygène à la condition que $R_1$ soit dans ce cas différent du groupement méthyle,
Z représente un atome d'hydrogène ou bien Z forme avec Y une liaison $\pi$,
T représente un atome d'hydrogène ou un groupement alkyle inférieur,
le terme inférieur indiquant que les groupements ainsi qualifiés comptent de 1 à 6 atomes de carbone, leurs énantiomères, diastéréoisomères et épimères, leurs sels d'ammonium quaternaire, leurs sels d'addition à un acide pharmaceutiquement acceptable.

2/ Composés selon la revendication 1, dans lesquels X et Y représentent simultanément un atome d'hydrogène, leurs énantiomères, diastéréoisomères et épimères, leurs sels d'ammonium quaternaire, leurs sels d'addition à un acide pharmaceutiquement acceptable.

3/ Composés selon la revendication 1, dans lesquels X représente un atome d'hydrogène et Y un groupement hydroxyle, leurs énantiomères, diastéréoisomères et épimères, leurs sels d'ammonium quaternaire, leurs sels d'addition à un acide pharmaceutiquement acceptable.

4/ Composés selon la revendication 1, dans lesquels X et Y simultanément représentent un atome d'oxygène, leurs énantiomères, diastéréoisomères et épimères, leurs sels d'ammonium quaternaire, leurs sels d'addition à un acide pharmaceutiquement acceptable.

5/ Composés selon la revendication 1, dans lesquels Y forme avec Z une liaison $\pi$, leurs énantiomères, diastéréoisomères et épimères, leurs sels d'ammonium quaternaire, leurs sels d'addition à un acide pharmaceutiquement acceptable.

6/ Composé selon l'une des revendications 1 et 2 qui est la méthyl - 3 (morpholino - 3 propyl) - 6 benzoxazolinone ainsi que ses sels d'addition à un acide pharmaceutiquement acceptable.

7/ Procédé de préparation des composés de formule (I) selon la revendication 1, caractérisé en ce que l'on utilise comme matière première un dérivé de formule (II) :

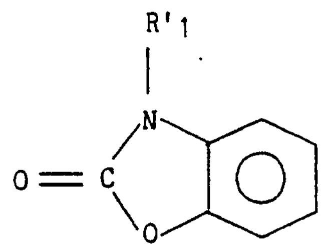

(II)

dans laquelle, $R'_1$ représente un atome d'hydrogène ou un groupement alkyle inférieur,
que l'on soumet à l'action d'un chlorure d'acide de formule (III) :

$Cl - CO - \underset{\displaystyle T}{\underset{|}{HC}} - CH_2 - A$ (III)

dans laquelle, T a la même signification que dans la formule (I), A représentant un atome d'halogène,
ou bien de l'anhydride d'acide correspondant,
en présence de chlorure d'aluminium en milieu de diméthyl formamide,
pour obtenir un dérivé de formule (IV) :

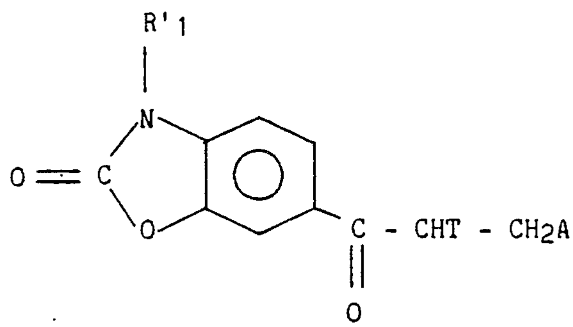

(IV)

dans laquelle $R'_1$, T et A ont la même signification que précédemment, qui, si on le désire, est soumis à réduction par un trialkylsilane en milieu acide, pour conduire à un dérivé de formule (V) :

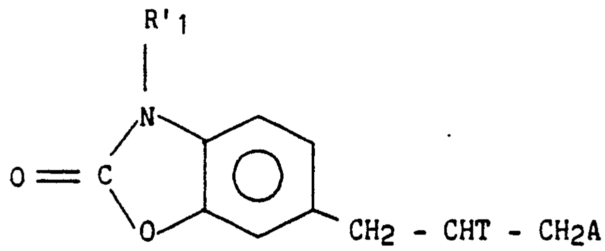

(V)

dans laquelle $R'_1$, T et A ont la même signification que précédemment, le dérivé de formule (IV), ou le dérivé de formule (V), selon la formule du dérivé de formule (I) que l'on souhaite obtenir, étant alors soumis à l'action d'une amine de formule (VI) :

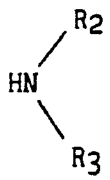

(VI)

dans laquelle $R_2$ et $R_3$ ont la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi, en présence d'un excès de l'amine choisie ou d'un capteur de l'hydracide formé tel que la triéthylamine, pour conduire à un dérivé de formule (I/A) :

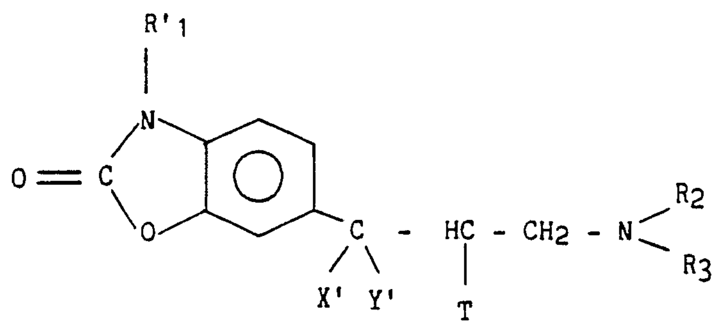

(I/A)

dans laquelle, selon que la matière première utilisée est un dérivé de formule (IV) ou (V), $X'$ et $Y'$ représentent ensemble un atome d'oxygène, ou bien X et Y représentent chacun simultanément un atome d'hydrogène, $R_1'$, $R_2$, $R_3$, T ayant la même signification que précédemment, qui, si on le désire, est salifié par un acide pharmaceutiquement acceptable ou qui peut, lorsque $X'$ et $Y'$ représentent ensemble un atome d'oxygène, si on le désire, être soumis,

* ou bien, à un agent d'hydrogénation choisi parmi un hydrure mixte de métal alcalin, comme par exemple le borohydrure de sodium, ou un cyanohydrure mixte de métal alcalin, comme le cyano borohydrure de sodium, préférentiellement en milieu d'alcool aliphatique inférieur, pour conduire à un dérivé de formule (I/B) - majoritairement sous la configuration thréo. lorsque T ne représente pas un atome d'hydrogène - :

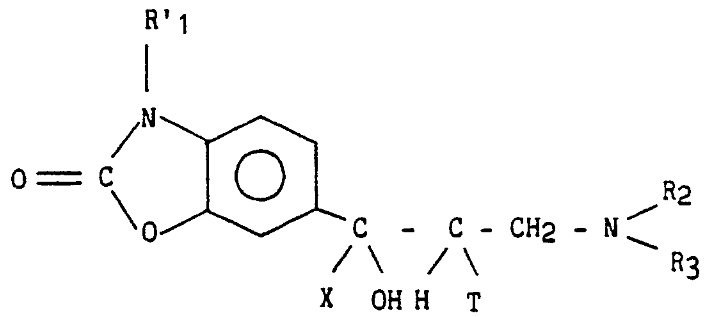

(I/B)

cas particulier des dérivés de formule (I), dans laquelle :
$R'_1$, $R_2$, $R_3$ et T ont la même signification que précédemment,
X représente un atome d'hydrogène,
Y un groupement hydroxyle et Z un atome d'hydrogène,
dont on sépare, si on le désire, les isomères et/ou que l'on salifie par un acide pharmaceutiquement acceptable,
* ou bien à hydrogénation catalytique, sous chauffage et pression dans un solvant choisi parmi alcool aliphatique inférieur ou dioxanne, pour conduire à un dérivé de formule (I/B) - essentiellement sous la configuration érythro lorsque T ne représente pas un atome d'hydrogène - dont on sépare, si on le désire, les isomères et que l'on salifie le cas échéant par un acide pharmaceutiquement. acceptable,
dérivé de formule (I/B) qui, quelque soit le procédé selon lequel il a été obtenu peut être, si on le désire, traité par un agent déshydratant,
préférentiellement choisi parmi les hydracides, pour conduire à un dérivé de formule (I/C) :

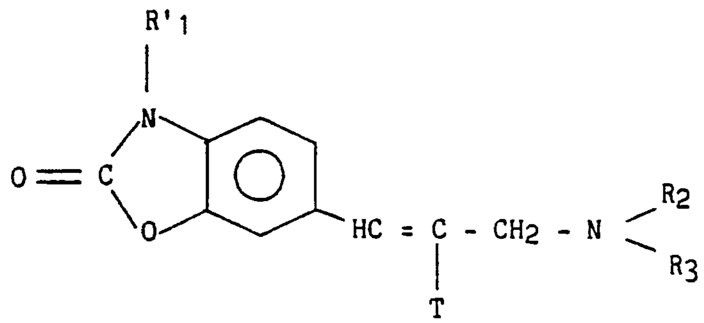

(I/C)

majoritairement sous forme d'isomère trans, cas particulier de dérivés de formule (1), dans laquelle :
$R'_1$, $R_2$, $R_3$ et T ont la même signification que précédemment,
X représente un atome d'hydrogène,
Z forme avec Y une liaison $\pi$,
dont, on sépare, si on le désire, les isomères cis-trans par une technique usuelle telle que la chromatographie sur colonne de silice ou la cristallisation,
et qui, si on le souhaite, peut être salifié par un acide pharmaceutiquement acceptable,
dérivé de formule (I/A), (I/B) ou (I/C),
- qui, lorsque $R'_1$ représente un atome d'hydrogèen, peut être traité en présence d'une base forte par un dérivé de formule $X-(CH_2)_n-OH$,
dans laquelle X représente un atome d'hydrogène et n est compris entre 1 et 6 pour conduire à un dérivé de formule (I) pour lequel R1 représente un groupement alkyle inférieur substitué par un groupement hydroxyle,
- dérivé de formule (I) que l'on peut traiter, si on le désire, par un agent d'alkylation classique tel que le sulfate de méthyle pour conduire à un sel d'ammonium quaternaire.

8/ Procédé de préparation des dérivés de formule (I) selon la revendication 1 pour lesquels $R_1$ représente un groupement alkyle inférieur caractérisé en ce que l'on acyle un dérivé de formule (II) :

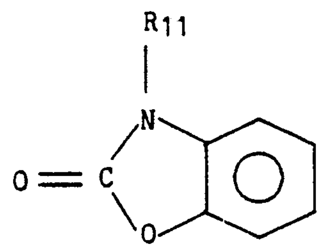

(II)

dans laquelle $R_{11}$ représente un groupement alkyle inférieur par un acide de formule (VII) :

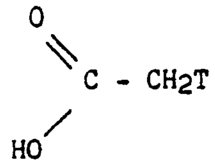

(VII)

dans laquelle T a la même signification que dans la formule (I) ou le chlorure ou l'anhydride de l'acide correspondant en présence d'acide polyphosphonique,
pour obtenir un dérivé de formule (VIII) :

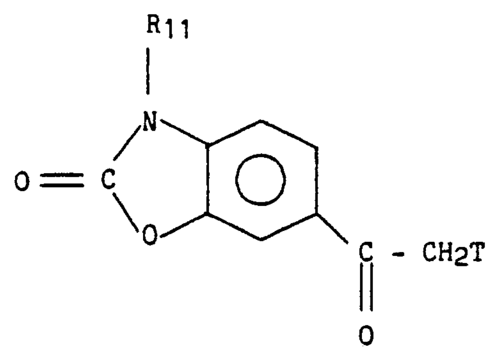

(VIII)

dans laquelle, $R_{11}$ représente un groupement alkyle inférieur, et T a la même signification que dans la formule (I),
que l'on traite ensuite,
* ou bien, en présence de trioxyméthylène et de l'amine de formule (VI) :

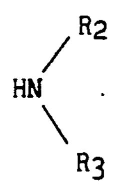

(VI)

dans laquelle, $R_2$ et $R_3$ ont la même signification que dans la formule (I),
pour obtenir un dérivé de formule (I/A1) :

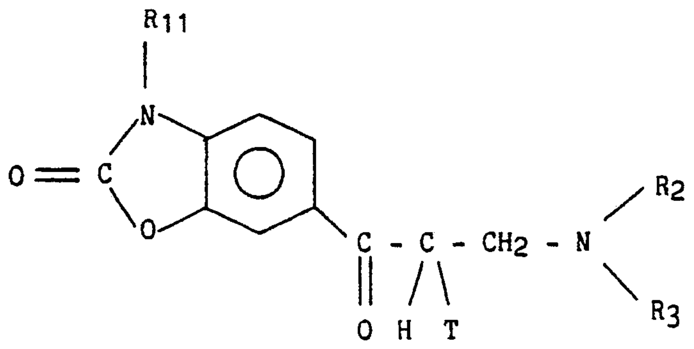

(I/A1)

cas particulier des dérivés de formules (I/A) et (I), dans laquelle :
$R_{11}$ représente un groupement alkyle inférieur, $R_2$, $R_3$ et T, ont la même signification que dans la formule (I),
X et Y représentent simultanément un atome d'oxygène et Z un atome d'hydrogène,
* ou bien par le bisdiméthylaminométhane en présence d'anhydride acétique pour obtenir un produit de formule générale (IX) :

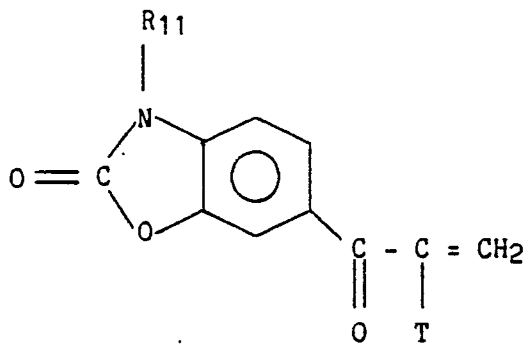

(IX)

dans laquelle :
T a la même signification que dans la formule (I) et $R_{11}$ représente un groupement alkyle inférieur,
que l'on traite par une amine de formule (VI), dans un solvant polaire à une température comprise entre la température ambiante et la température d'ébullition du milieu réactionnel,
pour conduire à un dérivé de formule (I/A1) précédemment défini,
qui, lorsque T ne représente pas un atome d'hydrogène peut être, si on le désire, séparé en ses isomères, que l'on salifie, si on le désire par un acide pharmaceutiquement acceptable et qui,
si on le souhaite, peut être soumis,
* ou bien préférentiellement en milieu d'alcool aliphatique inférieur à un agent d'hydrogénation préférentiellement un hydrure mixte de métal alcalin ou un cyanohydrure mixte de métal alcalin comme par exemple le borohydrure de sodium, ou le cyanoborohydrure de sodium, pour conduire à un dérivé de formule (I/B) majoritairement sous la configuration thréo (lorsque T ne représente pas un atome d'hydrogène) :

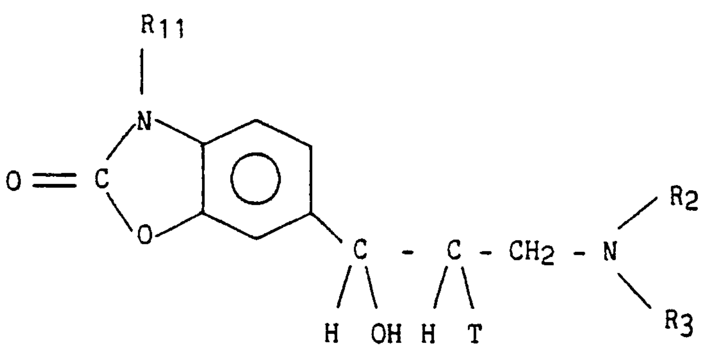

(I/B)

cas particulier des dérivés de formule (I), dans laquelle :
$R_{11}$, $R_2$, $R_3$ et T ont la même signification que dans les dérivés de formule (I),
X représente un atome d'hydrogène,
Y un groupement hydroxyle et Z un atome d'hydrogène,
dont on sépare, si on le désire, les isomères et que l'on peut salifier par un acide pharmaceutiquement acceptable,
* ou bien à hydrogénation catalytique dans un solvant choisi parmi alcool aliphatique inférieur ou dioxanne pour conduire à un dérivé de formule (I/B) essentiellement sous la configuration érythro -lorsque T ne représente pas un atome d'hydrogène - dont on sépare, si on le désire, les isomères et que l'on salifie le cas échéant par un acide pharmaceutiquement acceptable,
dérivé de formule (I/B) que, le cas échéant, on soumet à un agent déshydratant choisi préférentiellement parmi les hydracides pour conduire à un dérivé de formule (I/C), majoritairement sous forme d'isomères trans :

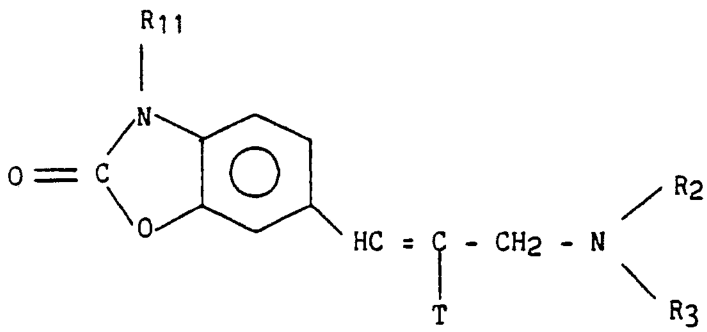

(I/C)

cas particulier de dérivés de formule (I), dans laquelle :
$R_{11}$, $R_2$, $R_3$ et T ont la même signification que dans les dérivés de formule (I),
X représente un atome d'hydrogène,
Z forme avec Y une liaison $\pi$,
dont, si on le désire, on sépare les isomères cis-trans par une technique usuelle telle que la chromatographie sur colonne de silice ou la cristallisation,
et que l'on salifie, si on le désire, par un acide pharmaceutiquement acceptable,
qui, si on le désire, est soumis à une réaction d'hydrogénation catalytique préférentiellement à température et pression ambiantes, et en présence de Nickel de Raney en milieu d'alcool aliphatique inférieur ou de dioxanne pour obtenir un dérivé de formule (I/D) :

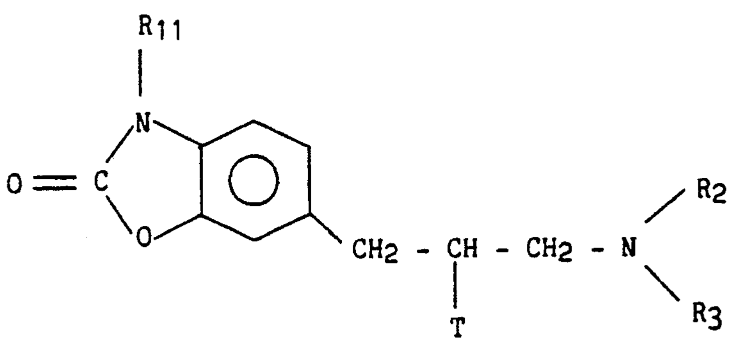

(I/D)

dans laquelle :
$R_{11}$, $R_2$, $R_3$ et T ont la même signification que précédemment,
X, Y et Z représentent chacun simultanément un atome d'hydrogène.
dont on sépare le cas échéant les isomères lorsque T ne représente pas un atome d'hydrogène,
et, que l'on salifie éventuellement par un acide pharmaceutiquement acceptable, ou que l'on transforme en sel d'ammonium quaternaire par action d'un agent d'alkylation comme indiqué précédemment.

9/ Procédé de préparation des dérivés de formule (I/D), selon la revendication 8 à partir des dérivés de

formule (IX) :

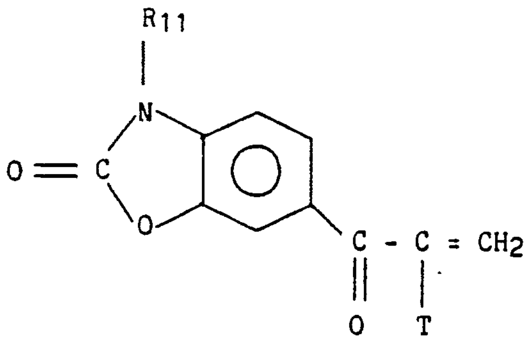

(IX)

dans laquelle :
$R_{11}$ et T ont la même signification que dans la formule (I),
que l'on traite par un hydracide pour obtenir un dérivé de formule (IV) :

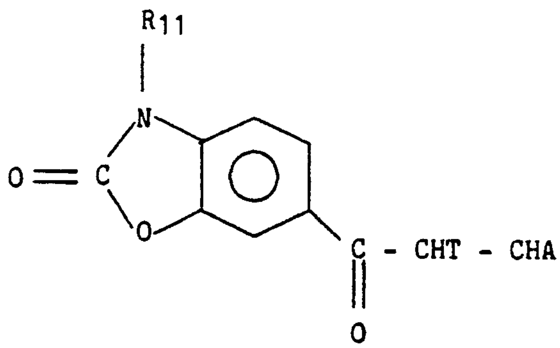

(IV)

dans laquelle $R_{11}$ et T ont la même signification que précédemment et A la même signification que dans la formule (III),
dont on sépare, si on le désire, les isomères lorsque T ne représente pas un atome d'hydrogène,
que l'on soumet à réduction par un trialkylsilane en milieu acide pour conduire à un dérivé de formule (V) :

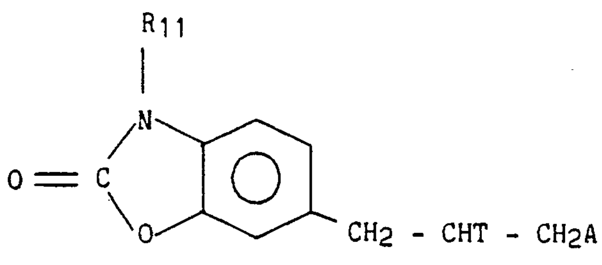

(V)

dans laquelle $R_{11}$ et T ont la même signification que précédemment et A la même signification que dans la formule (III),
que l'on soumet à l'action d'une amine de formule (VI) :

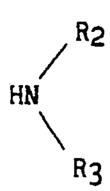

(VI)

dans laquelle $R_2$ et $R_3$ ont a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi en présence d'un excès de l'amine choisie ou d'un capteur de l'hydracide formé,
pour conduire à un dérivé de formule (I/D) selon la revendication 8 dont on sépare les isomères si on le désire et, que l'on salifie si on le désire par un acide pharmaceutiquement acceptable ou que l'on transforme en sel d'ammonium quaternaire par action d'un agent d'alkylation.

10/ Composition pharmaceutique contenant comme principe actif au moins un composé selon l'une des revendications 1 à 6 en combinaison avec un ou plusieurs excipients ou véhicules inertes, non-toxiques, pharmaceutiquement acceptables.

11/ Composition pharmaceutique selon la revendication 10 contenant au moins un principe actif selon l'une des revendications 1 à 7 utilisable dans le traitement d'algies, des troubles du système nerveux central ou de l'hypertension artérielle.

Revendications pour l'Etat contractant suivant:ES

1/ Procédé de préparation de composés de formule générale (I) :

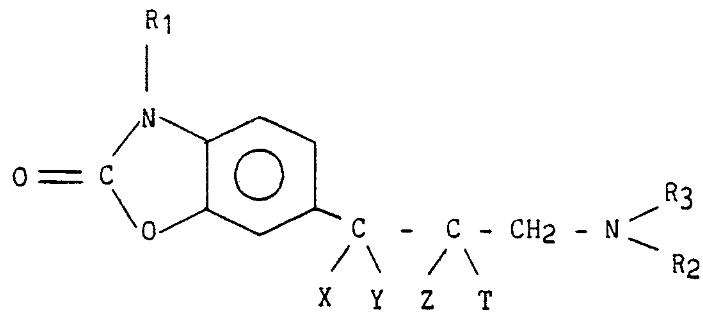

(I)

dans laquelle :
$R_1$ représente un atome d'hydrogène ou groupement alkyle inférieur éventuellement substitué par un groupement hydroxyle,
$R_2$ et $R_3$ identiques ou différents représentent :
- un atome d'hydrogène,
- un groupement alkyle inférieur linéaire ou ramifié,
- un groupement alkényle inférieur linéaire ou ramifié,
- un groupement aryle ou alkyle inférieur aryle dans lesquels la partie aryle est éventuellement substituée par un ou plusieurs atomes d'halogène ou groupements alkyle inférieurs éventuellement substitués par un ou plusieurs atomes d'halogène ou groupements alkoxy inférieurs,
ou bien, $R_2$ et $R_3$ constituent avec l'atome d'azote auquel ils sont liés un système hétérocyclique, mono ou bicyclique, saturé ou non contenant un, deux ou trois hétéroatomes par cycle choisis parmi azote, oxygène ou soufre, substitué ou non par un halogène, un groupement alkyle inférieur, alkoxy inférieur ou aryle éventuellement substitué par un ou plusieurs atomes d'halogène, à la condition que $R_2$ et $R_3$ ne constituent pas avec l'atome auquel ils sont attachés un système aryl - 1 pipérazénique,
X représente un atome d'hydrogène,
Y représente un atome d'hydrogène ou un groupement hydroxyle,
ou bien X et Y représentent ensemble un atome d'oxygène à la condition que $R_1$ soit dans ce cas différent du groupement méthyle,
Z représente un atome d'hydrogène ou bien Z forme avec Y une liaison $\pi$,
T représente un atome d'hydrogène ou un groupement alkyle inférieur,
le terme inférieur indiquant que les groupements ainsi qualifiés comptent de 1 à 6 atomes de carbone,
leurs énantiomères, diastéréoisomères et épimères, leurs sels d'ammonium quaternaire, leurs sels d'addition à un acide pharmaceutiquement acceptable,
caractérisé 1) ou bien en ce que l'on utilise comme matière première un dérivé de formule (II) :

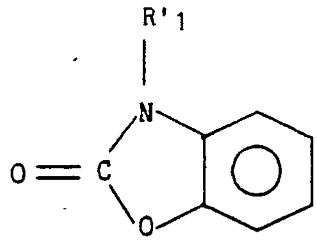

(II)

dans laquelle, $R'_1$ représente un atome d'hydrogène ou un groupement alkyle inférieur, que l'on soumet à l'action d'un chlorure d'acide de formule (III) :

Cl - CO - HC - $CH_2$ - A (III)
T

dans laquelle, T a la même signification que dans la formule (I), A représentant un atome d'halogène, ou bien de l'anhydride d'acide correspondant, en présence de chlorure d'aluminium en milieu de diméthyl formamide, pour obtenir un dérivé de formule (IV) :

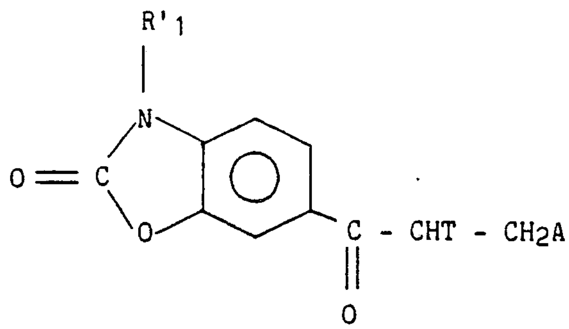

(IV)

dans laquelle $R'_1$, T et A ont la même signification que précédemment, qui, si on le désire, est soumis à réduction par un trialkylsilane en milieu acide, pour conduire à un dérivé de formule (V) :

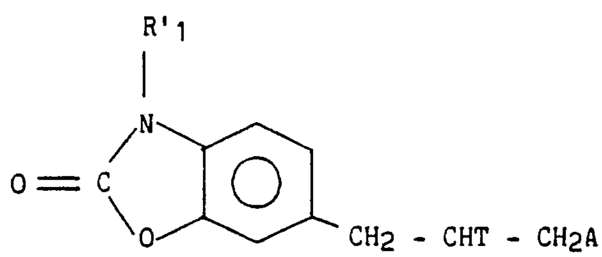

(V)

dans laquelle $R'_1$, T et A ont la même signification que précédemment, le dérivé de formule (IV), ou le dérivé de formule (V), selon la formule du dérivé de formule (I) que l'on souhaite obtenir, étant alors soumis à l'action d'une amine de formule (VI) :

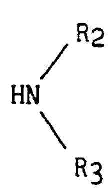

(VI)

dans laquelle $R_2$ et $R_3$ ont la même signification que dans la formule (I), dans un solvant préférentiellement choisi parmi acétone, acétonitrile, acétate d'éthyle, alcool aliphatique

inférieur, dioxanne, benzène, toluène, à une température comprise entre la température ambiante et la température d'ébullition du solvant choisi, en présence d'un excès de l'amine choisie ou d'un capteur de l'hydracide formé tel que la triéthylamine,
pour conduire à un dérivé de formule (I/A) :

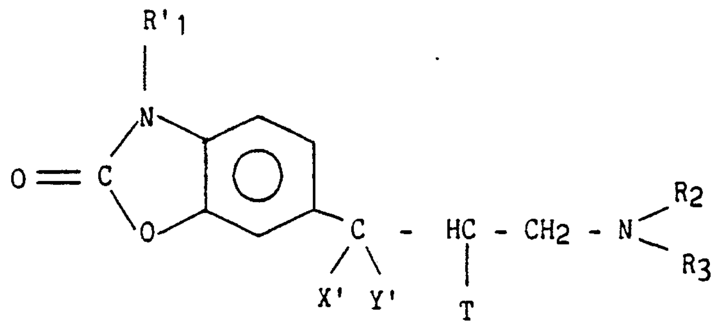

(I/A)

dans laquelle, selon que la matière première utilisée est un dérivé de formule (IV) ou (V),
X′ et Y′ représentent ensemble un atome d'oxygène, ou bien X et Y représentent chacun simultanément un atome d'hydrogène,
$R_1'$, $R_2$, $R_3$, T ayant la même signification que précédemment,
qui, si on le désire, est salifié par un acide pharmaceutiquement acceptable ou qui peut, lorsque X′ et Y′ représentent ensemble un atome d'oxygène, si on le désire, être soumis,
* ou bien, à un agent d'hydrogénation choisi parmi un hydrure mixte de métal alcalin, comme par exemple le borohydrure de sodium, ou un cyanohydrure mixte de métal alcalin, comme le cyano borohydrure de sodium, préférentiellement en milieu d'alcool aliphatique inférieur, pour conduire à un dérivé de formule (I/B) - majoritairement sous la configuration thréo lorsque T ne représente pas un atome d'hydrogène - :

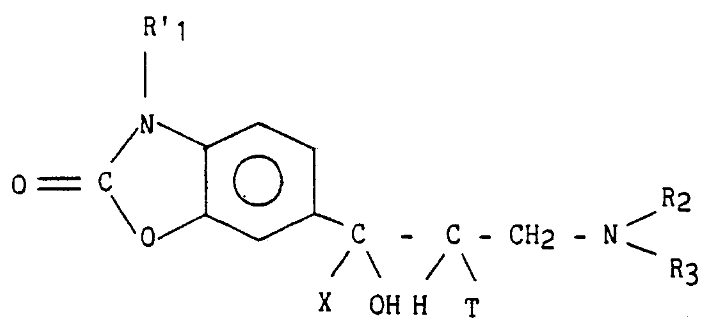

(I/B)

cas particulier des dérivés de formule (I), dans laquelle :
$R'_1$, $R_2$, $R_3$ et T ont la même signification que précédemment,
X représente un atome d'hydrogène,
Y un groupement hydroxyle et Z un atome d'hydrogène,
dont on sépare, si on le désire, les isomères et/ou que l'on salifie par un acide pharmaceutiquement acceptable,
* ou bien à hydrogénation catalytique, sous chauffage et pression dans un solvant choisi parmi alcool aliphatique inférieur ou dioxanne, pour conduire à un dérivé de formule (I/B) - essentiellement sous la configuration érythro lorsque T ne représente pas un atome d'hydrogène - dont on sépare, si on le désire, les isomères et que l'on salifie le cas échéant par un acide pharmaceutiquement acceptable,
dérivé de formule (I/B) qui, quelque soit le procédé selon lequel il a été obtenu peut être, si on le désire, traité par un agent déshydratant,
préférentiellement choisi parmi les hydracides, pour conduire à un dérivé de formule (I/C) :

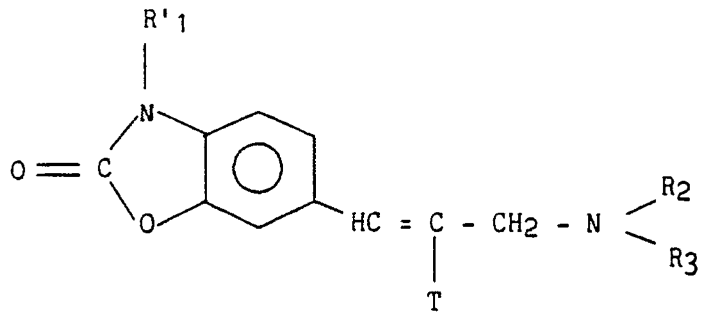

(I/C)

majoritairement sous forme d'isomère trans, cas particulier de dérivés de formule (I), dans laquelle :
$R'_1$, $R_2$, $R_3$ et T ont la même signification que précédemment,
X représente un atome d'hydrogène,
Z forme avec Y une liaison $\pi$,
dont, on sépare, si on le désire, les isomères cis-trans par une technique usuelle telle que la chromatographie sur colonne de silice ou la cristallisation,
et qui, si on le souhaite, peut être salifié par un acide pharmaceutiquement acceptable,
dérivé de formule (I/A), (I/B) ou (I/C),
- qui, lorsque $R'_1$ représente un atome d'hydrogèen, peut être traité en présence d'une base forte par un dérivé de formule $X-(CH_2)_n-OH$,
dans laquelle X représente un atome d'hydrogène et n est compris entre 1 et 6 pour conduire à un dérivé de formule (I) pour lequel R1 représente un groupement alkyle inférieur substitué par un groupement hydroxyle,
- dérivé de formule (I) que l'on peut traiter, si on le désire, par un agent d'alkylation classique tel que le sulfate de méthyle pour conduire à un sel d'ammonium quaternaire,
ou bien, 2) en ce que l'on acyle un dérivé de formule (II) lorsque R1 représente un groupement alkyle inférieur :

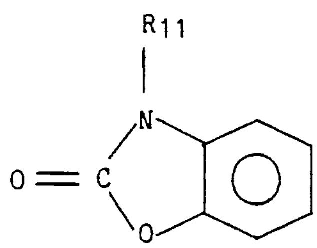

(II)

dans laquelle $R_{11}$ représente un groupement alkyle inférieur par un acide de formule (VII) :

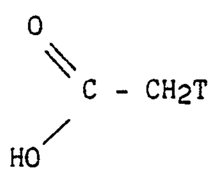

(VII)

dans laquelle T a la même signification que dans la formule (I) ou le chlorure ou l'anhydride de l'acide correspondant en présence d'acide polyphosphonique,
pour obtenir un dérivé de formule (VIII) :

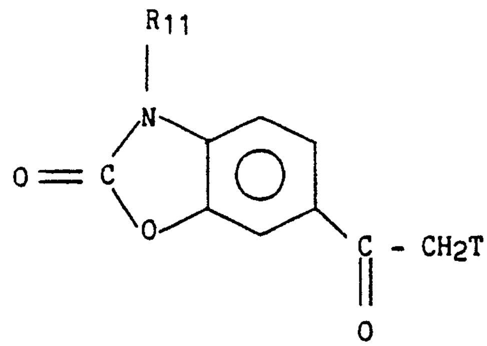

(VIII)

dans laquelle, $R_{11}$ représente un groupement alkyle inférieur, et T a la même signification que dans la formule (I),
que l'on traite ensuite,
* ou bien, en présence de trioxyméthylène et de l'amine de formule (VI) :

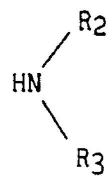

(VI)

dans laquelle, $R_2$ et $R_3$ ont la même signification que dans la formule (I),
pour obtenir un dérivé de formule (I/A1) :

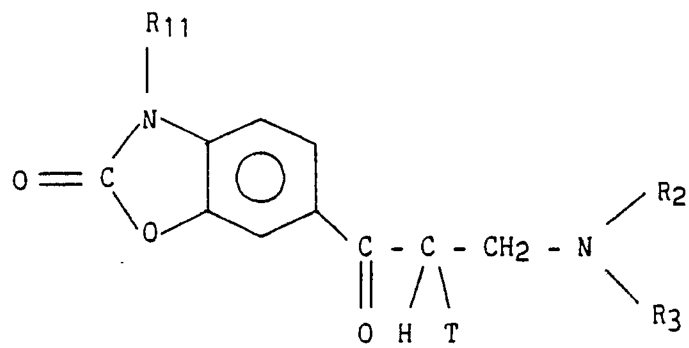

(I/A1)

cas particulier des dérivés de formules (I/A) et (I), dans laquelle :
$R_{11}$ représente un groupement alkyle inférieur, $R_2$, $R_3$ et T, ont la même signification que dans la formule (I),
X et Y représentent simultanément un atome d'oxygène et Z un atome d'hydrogène,
* ou bien par le bisdiméthylaminométhane en présence d'anhydride acétique pour obtenir un produit de formule générale (IX) :

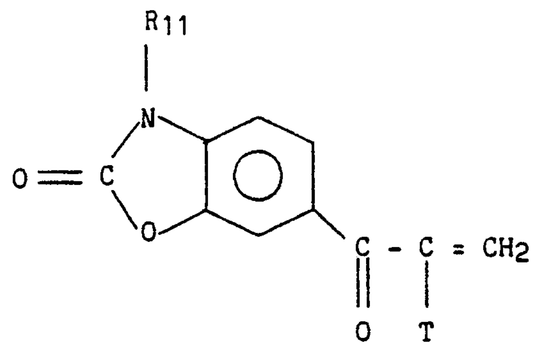

(IX)

dans laquelle :
T a la même signification que dans la formule (I) et $R_{11}$ représente un groupement alkyle inférieur,
que l'on traite par une amine de formule (VI), dans un solvant polaire à une température comprise entre la température ambiante et la température d'ébullition du milieu réactionnel,
pour conduire à un dérivé de formule (I/A1) précédemment défini,
qui, lorsque T ne représente pas un atome d'hydrogêne peut être, si on le désire, séparé en ses isomères,
que l'on salifie, si on le désire par un acide pharmaceutiquement acceptable et qui,
si on le souhaite, peut être soumis,
* ou bien préférentiellement en milieu d'alcool aliphatique inférieur à un agent d'hydrogénation préférentiellement un hydrure mixte de métal alcalin ou un cyanohydrure mixte de métal alcalin comme par exemple le borohydrure de sodium, ou le cyanoborohydrure de sodium,
pour conduire à un dérivé de formule (I/B) majoritairement sous la configuration thréo (lorsque T ne représente pas un atome d'hydrogène) :

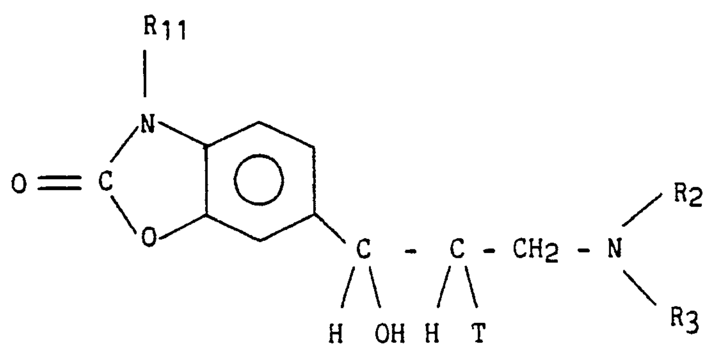

(I/B)

cas particulier des dérivés de formule (I), dans laquelle :
$R_{11}$, $R_2$, $R_3$ et T ont la même signification que dans les dérivés de formule (I),
X représente un atome d'hydrogène,
Y un groupement hydroxyle et Z un atome d'hydrogène,
dont on sépare, si on le désire, les isomères et que l'on peut salifier par un acide pharmaceutiquement acceptable,
* ou bien à hydrogénation catalytique dans un solvant choisi parmi alcool aliphatique inférieur ou dioxanne pour conduire à un dérivé de formule (I/B) essentiellement sous la configuration érythro -lorsque T ne représente pas un atome d'hydrogène - dont on sépare, si on le désire, les isomères et que l'on salifie le cas échéant par un acide pharmaceutiquement acceptable,
dérivé de formule (I/B) que, le cas échéant, on soumet à un agent déshydratant choisi préférentiellement parmi les hydracides pour conduire à un dérivé de formule (I/C), majoritairement sous forme d'isomères trans :

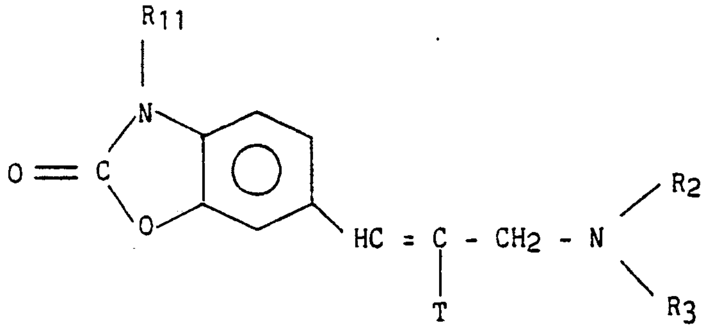

(I/C)

cas particulier de dérivés de formule (I), dans laquelle :
$R_{11}$, $R_2$, $R_3$ et T ont la même signification que dans les dérivés de formule (I),
X représente un atome d'hydrogène,
Z forme avec Y une liaison $\pi$,
dont, si on le désire, on sépare les isomères cis-trans par une technique usuelle telle que la chromatographie sur colonne de silice ou la cristallisation,
et que l'on salifie, si on le désire, par un acide pharmaceutiquement acceptable,
qui, si on le désire, est soumis à une réaction d'hydrogénation catalytique préférentiellement à température et pression ambiantes, et en présence de Nickel de Raney en milieu d'alcool aliphatique inférieur ou de dioxanne pour obtenir un dérivé de formule (I/D) :

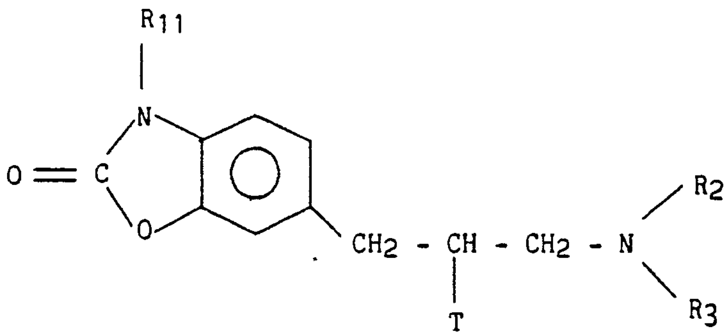

(I/D)

dans laquelle :
$R_{11}$, $R_2$, $R_3$ et T ont la même signification que précédemment,
X, Y et Z représentent chacun simultanément un atome d'hydrogène.
dont on sépare le cas échéant les isomères lorsque T ne représente pas un atome d'hydrogène,
et, que l'on salifie éventuellement par un acide pharmaceutiquement acceptable, ou que l'on transforme en sel d'ammonium quaternaire par action d'un agent d'alkylation comme indiqué précédemment.

2/ Procédé de préparation des dérivés de formule (I/D), selon la revendication 1 à partir des dérivés de formule (IX) :

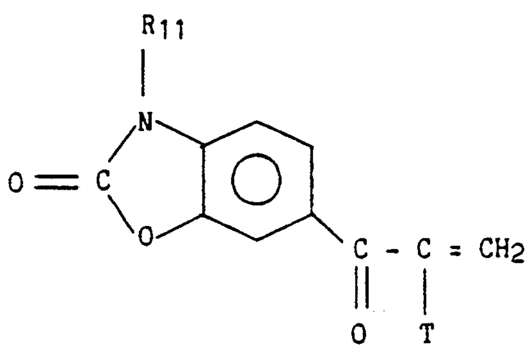

(IX)

dans laquelle :
$R_{11}$ et T ont la même signification que dans la formule (I),
que l'on traite par un hydracide pour obtenir un dérivé de formula (IV) :

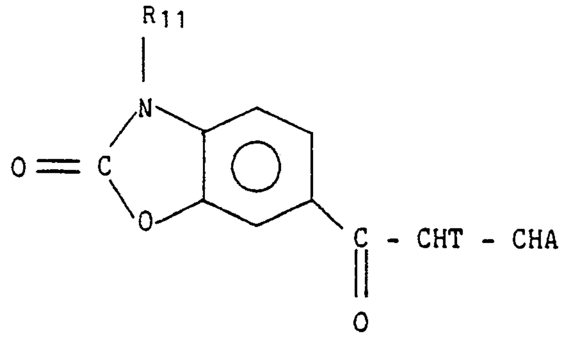

(IV)

dans laquelle $R_{11}$ et T ont la même signification que précédemment et A la même signification que dans la formule (III),
dont on sépare, si on le désire, les isomères lorsque T ne représente pas un atome d'hydrogène,
que l'on soumet à réduction par un trialkylsilane en milieu acide pour conduire à un dérivé de formule (V) :

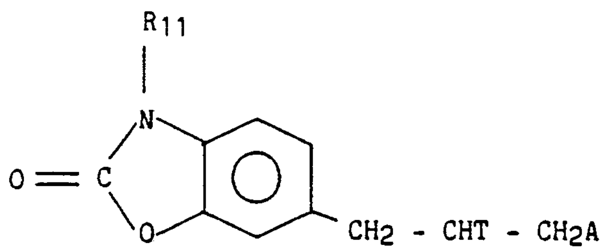

(V)

dans laquelle $R_{11}$ et T ont la même signification que précédemment et A la même signification que dans la formule (III),
que l'on soumet à l'action d'une amine de formule (VI) :

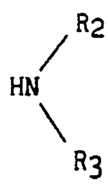

(VI)

dans laquelle $R_2$ et $R_3$ ont a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi en présence d'un excès de l'amine choisie ou d'un capteur de l'hydracide formé,
pour conduire à un dérivé de formule (I/D) selon la revendication 1 dont on sépare les isomères si on le désire et, que l'on salifie si on le désire par un acide pharmaceutiquement acceptable ou que l'on transforme en sel d'ammonium quaternaire par action d'un agent d'alkylation.

3/ Procédé de préparation selon la revendication 1 de composés de formules (I), dans lesquels X et Y représentent simultanément un atome d'hydrogène, leurs énantiomères, diastéréoisomères et épimères, leurs sels d'ammonium quaternaire, leurs sels d'addition à un acide pharmaceutiquement acceptable.

4/ Procédé de préparation selon la revendication 1 de composés de formule (I), dans lesquels X représente un atome d'hydrogène et Y un groupement hydroxyle, leurs énantiomères, diastéréoisomères et épimères, leurs sels d'ammonium quaternaire, leurs sels d'addition à un acide pharmaceutiquement acceptable.

5/ Procédé de préparation selon la revendication 1 de composés de formule (I), dans lesquels X et Y

simultanément représentent un atome d'oxygène, leurs énantiomères, diastéréoisomères et épimères, leurs sels d'ammonium quaternaire, leurs sels d'addition à un acide pharmaceutiquement acceptable.

6/ Procédé de préparation selon la revendication 1 de composés de formule (I), dans lesquels Y forme avec Z une liaison $\pi$, leurs énantiomères, diastéréoisomères et épimères, leurs sels d'ammonium quaternaire, leurs sels d'addition à un acide pharmaceutiquement acceptable.

7/ Procédé de préparation selon l'une des revendications 1 et 2 de composés de formule (I) qui est la méthyl - 3 (morpholino - 3 propyl) -6 benzoxazolinone ainsi que ses sels d'addition à un acide pharmaceutiquement acceptable.

Revendications pour l'Etat contractant suivant:GR

1/ Procédé de préparation de composés de formule générale (I) :

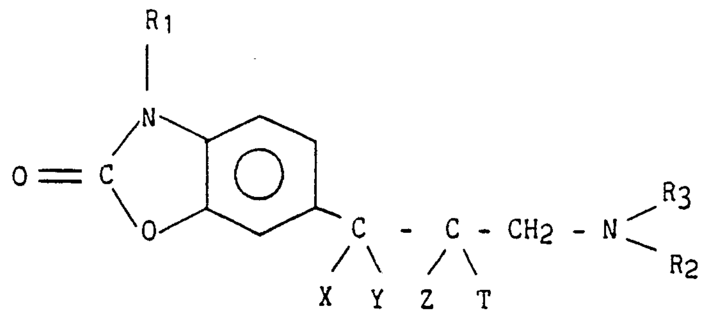

(I)

dans laquelle :
$R_1$ représente un atome d'hydrogène ou groupement alkyle inférieur éventuellement substitué par un groupement hydroxyle,
$R_2$ et $R_3$ identiques ou différents représentent :
- un atome d'hydrogène,
- un groupement alkyle inférieur linéaire ou ramifié,
- un groupement alkényle inférieur linéaire ou ramifié,
- un groupement aryle ou alkyle inférieur aryle dans lesquels la partie aryle est éventuellement substituée par un ou plusieurs atomes d'halogène ou groupements alkyle inférieurs éventuellement substitués par un ou plusieurs atomes d'halogène ou groupements alkoxy inférieurs,
ou bien, $R_2$ et $R_3$ constituent avec l'atome d'azote auquel ils sont liés un système hétérocyclique, mono ou bicyclique, saturé ou non contenant un, deux ou trois hétéroatomes par cycle choisis parmi azote, oxygène ou soufre, substitué ou non par un halogène, un groupement alkyle inférieur, alkoxy inférieur ou aryle éventuellement substitué par un ou plusieurs atomes d'halogène, à la condition que $R_2$ et $R_3$ ne constituent pas avec l'atome auquel ils sont attachés un système aryl - 1 pipérazénique,
X représente un atome d'hydrogène,
Y représente un atome d'hydrogène ou un groupement hydroxyle,
ou bien X et Y représentent ensemble un atome d'oxygène à la condition que $R_1$ soit dans ce cas différent du groupement méthyle,
Z représente un atome d'hydrogène ou bien Z forme avec Y une liaison $\pi$,
T représente un atome d'hydrogène ou un groupement alkyle inférieur,
le terme inférieur indiquant que les groupements ainsi qualifiés comptent de 1 à 6 atomes de carbone,
leurs énantiomères, diastéréoisomères et épimères, leurs sels d'ammonium quaternaire, leurs sels d'addition à un acide pharmaceutiquement acceptable,
caractérisé 1) ou bien en ce que l'on utilise comme matière première un dérivé de formule (II) :

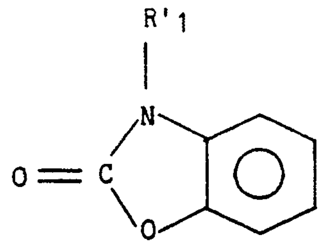

(II)

dans laquelle, $R'_1$ représente un atome d'hydrogène ou un groupement alkyle inférieur, que l'on soumet à l'action d'un chlorure d'acide de formule (III) :

$Cl - CO - \underset{\substack{|\\ T}}{HC} - CH_2 - A$ (III)

dans laquelle, T a la même signification que dans la formule (I), A représentant un atome d'halogène, ou bien de l'anhydride d'acide correspondant, en présence de chlorure d'aluminium en milieu de diméthyl formamide, pour obtenir un dérivé de formule (IV) :

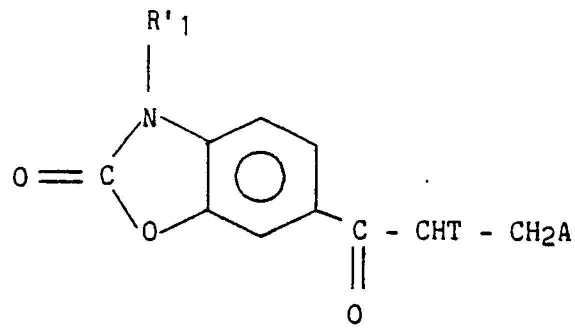

(IV)

dans laquelle $R'_1$, T et A ont la même signification que précédemment, qui, si on le désire, est soumis à réduction par un trialkylsilane en milieu acide, pour conduire à un dérivé de formule (V) :

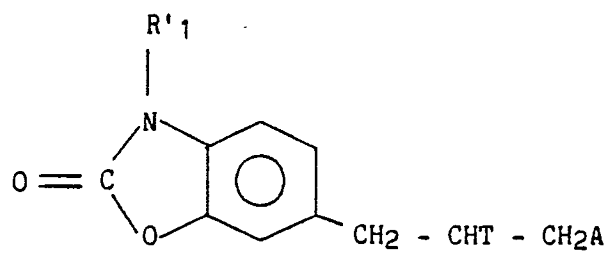

(V)

dans laquelle $R'_1$, T et A ont la même signification que précédemment, le dérivé de formule (IV), ou le dérivé de formule (V), selon la formule du dérivé de formule (I) que l'on souhaite obtenir, étant alors soumis à l'action d'une amine de formule (VI) :

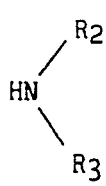

(VI)

dans laquelle $R_2$ et $R_3$ ont la même signification que dans la formule (I),

dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi, en présence d'un excès de l'amine choisie ou d'un capteur de l'hydracide formé tel que la triéthylamine,
pour conduire à un dérivé de formule (I/A) :

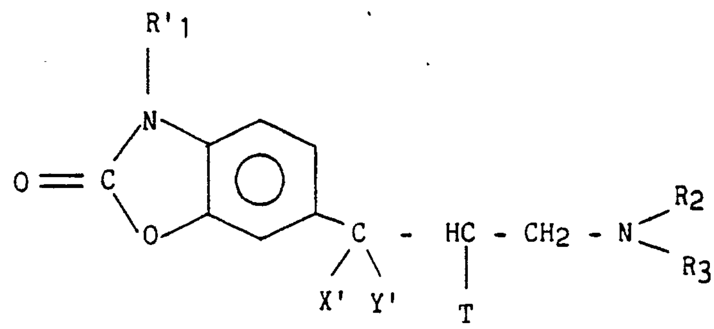

(I/A)

dans laquelle, selon que la matière première utilisée est un dérivé de formule (IV) ou (V),
X′ et Y′ représentent ensemble un atome d'oxygène, ou bien X et Y représentent chacun simultanément un atome d'hydrogène,
$R_1'$, $R_2$, $R_3$, T ayant la même signification que précédemment,
qui, si on le désire, est salifié par un acide pharmaceutiquement acceptable ou qui peut, lorsque X′ et Y′ représentent ensemble un atome d'oxygène, si on le désire, être soumis,
* ou bien, à un agent d'hydrogénation choisi parmi un hydrure mixte de métal alcalin, comme par exemple le borohydrure de sodium, ou un cyanohydrure mixte de métal alcalin, comme le cyano borohydrure de sodium, préférentiellement en milieu d'alcool aliphatique inférieur, pour conduire à un dérivé de formule (I/B) - majoritairement sous la configuration thréo lorsque T ne représente pas un atome d'hydrogène - :

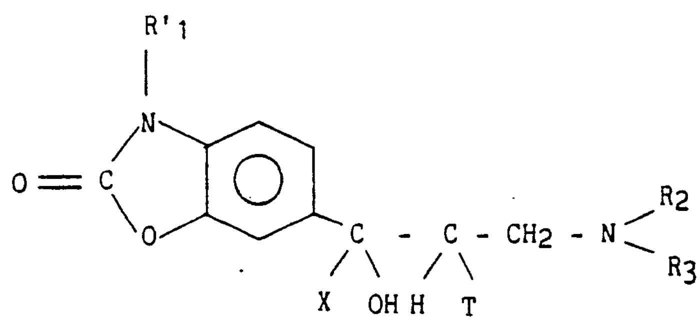

(I/B)

cas particulier des dérivés de formule (I), dans laquelle :
$R'_1$, $R_2$, $R_3$ et T ont la même signification que précédemment,
X représente un atome d'hydrogène,
Y un groupement hydroxyle et Z un atome d'hydrogène,
dont on sépare, si on le désire, les isomères et/ou que l'on salifie par un acide pharmaceutiquement acceptable,
* ou bien à hydrogénation catalytique, sous chauffage et pression dans un solvant choisi parmi alcool aliphatique inférieur ou dioxanne, pour conduire à un dérivé de formule (I/B) - essentiellement sous la configuration érythro lorsque T ne représente pas un atome d'hydrogène - dont on sépare, si on le désire, les isomères et que l'on salifie le cas échéant par un acide pharmaceutiquement acceptable,
dérivé de formule (I/B) qui, quelque soit le procédé selon lequel il a été obtenu peut être, si on le désire, traité par un agent déshydratant,
préférentiellement choisi parmi les hydracides, pour conduire à un dérivé de formule (I/C) :

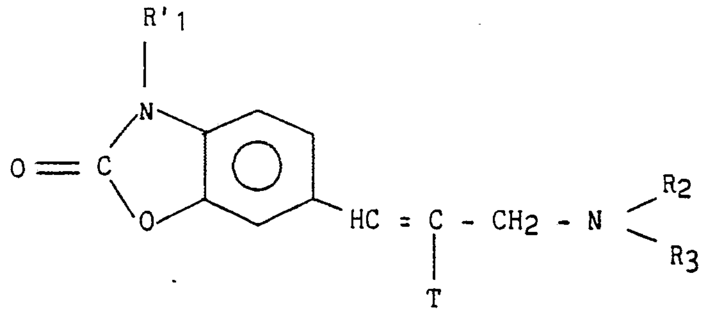

(I/C)

majoritairement sous forme d'isomère trans, cas particulier de dérivés de formule (I), dans laquelle :
$R'_1$, $R_2$, $R_3$ et T ont la même signification que précédemment,
X représente un atome d'hydrogène,
Z forme avec Y une liaison $\pi$,
dont, on sépare, si on le désire, les isomères cis-trans par une technique usuelle telle que la chromatographie sur colonne de silice ou la cristallisation,
et qui, si on le souhaite, peut être salifié par un acide pharmaceutiquement acceptable,
dérivé de formule (I/A), (I/B) ou (I/C),
- qui, lorsque $R'_1$ représente un atome d'hydrogèen, peut être traité en présence d'une base forte par un dérivé de formule $X\text{-}(CH_2)_n\text{-}OH$,
dans laquelle X représente un atome d'hydrogène et n est compris entre 1 et 6 pour conduire à un dérivé de formule (I) pour lequel R1 représente un groupement alkyle inférieur substitué par un groupement hydroxyle,
- dérivé de formule (I) que l'on peut traiter, si on le désire, par un agent d'alkylation classique tel que le sulfate de méthyle pour conduire à un sel d'ammonium quaternaire,
ou bien, 2) en ce que l'on acyle un dérivé de formule (II) lorsque R1 représente un groupement alkyle inférieur :

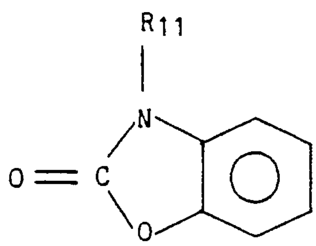

(II)

dans laquelle $R_{11}$ représente un groupement alkyle inférieur par un acide de formule (VII) :

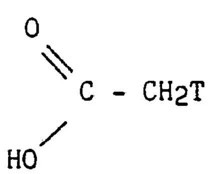

(VII)

dans laquelle T a la même signification que dans la formule (I) ou le chlorure ou l'anhydride de l'acide correspondant en présence d'acide polyphosphonique,
pour obtenir un dérivé de formule (VIII) :

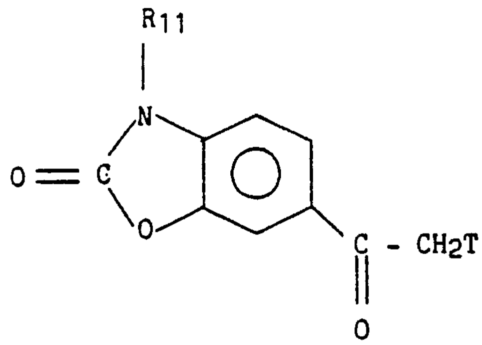

(VIII)

dans laquelle, $R_{11}$ représente un groupement alkyle inférieur, et T a la même signification que dans la formule (I),
que l'on traite ensuite,
* ou bien, en présence de trioxyméthylène et de l'amine de formule (VI) :

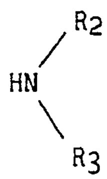

(VI)

dans laquelle, $R_2$ et $R_3$ ont la même signification que dans la formule (I),
pour obtenir un dérivé de formule (I/A1) :

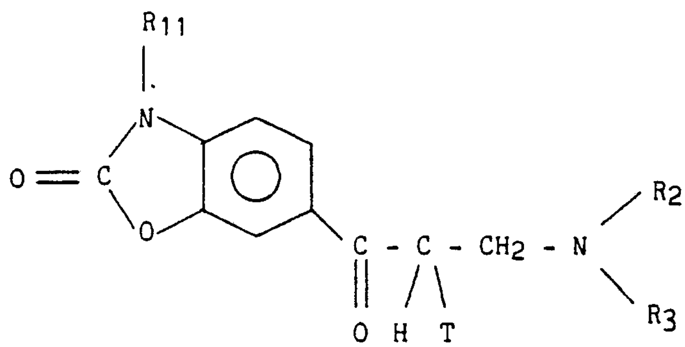

(I/A1)

cas particulier des dérivés de formules (I/A) et (I), dans laquelle :
$R_{11}$ représente un groupement alkyle inférieur, $R_2$, $R_3$ et T, ont la même signification que dans la formule (I),
X et Y représentent simultanément un atome d'oxygène et Z un atome d'hydrogène,
* ou bien par le bisdiméthylaminométhane en présence d'anhydride acétique pour obtenir un produit de formule générale (IX) :

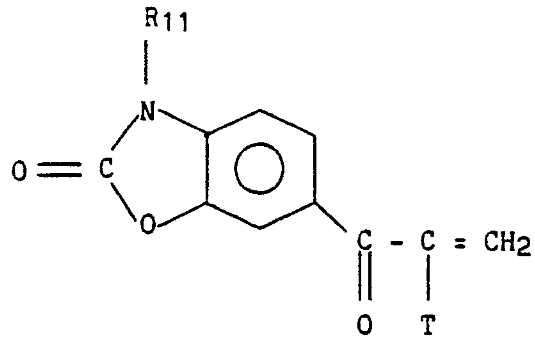

(IX)

dans laquelle :
T a la même signification que dans la formule (I) et $R_{11}$ représente un groupement alkyle inférieur, que l'on traite par une amine de formule (VI), dans un solvant polaire à une température comprise entre la température ambiante et la température d'ébullition du milieu réactionnel,
pour conduire à un dérivé de formule (I/A1) précédemment défini,
qui, lorsque T ne représente pas un atome d'hydrogène peut être, si on le désire, séparé en ses isomères, que l'on salifie, si on le désire par un acide pharmaceutiquement acceptable et qui,
si on le souhaite, peut être soumis,
* ou bien préférentiellement en milieu d'alcool aliphatique inférieur à un agent d'hydrogénation préférentiellement un hydrure mixte de métal alcalin ou un cyanohydrure mixte de métal alcalin comme par exemple le borohydrure de sodium, ou le cyanoborohydrure de sodium,
pour conduire à un dérivé de formule (I/B) majoritairement sous la configuration thréo (lorsque T ne représente pas un atome d'hydrogène) :

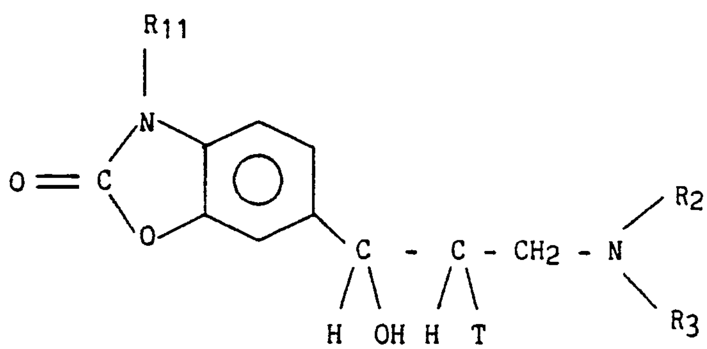

(I/B)

cas particulier des dérivés de formule (I), dans laquelle :
$R_{11}$, $R_2$, $R_3$ et T ont la même signification que dans les dérivés de formule (I),
X représente un atome d'hydrogène,
Y un groupement hydroxyle et Z un atome d'hydrogène,
dont on sépare, si on le désire, les isomères et que l'on peut salifier par un acide pharmaceutiquement acceptable,
* ou bien à hydrogénation catalytique dans un solvant choisi parmi alcool aliphatique inférieur ou dioxanne pour conduire à un dérivé de formule (I/B) essentiellement sous la configuration érythro -lorsque T ne représente pas un atome d'hydrogène - dont on sépare, si on le désire, les isomères et que l'on salifie le cas échéant par un acide pharmaceutiquement acceptable,
dérivé de formule (I/B) que, le cas échéant, on soumet à un agent déshydratant choisi préférentiellement parmi les hydracides pour conduire à un dérivé de formule (I/C), majoritairement sous forme d'isomères trans :

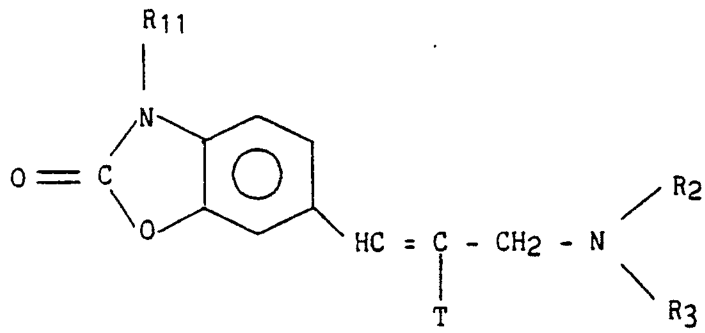

(I/C)

cas particulier de dérivés de formule (I), dans laquelle :
$R_{11}$, $R_2$, $R_3$ et T ont la même signification que dans les dérivés de formule (I),
X représente un atome d'hydrogène,
Z forme avec Y une liaison $\pi$,
dont, si on le désire, on sépare les isomères cis-trans par une technique usuelle telle que la chromatographie sur colonne de silice ou la cristallisation,
et que l'on salifie, si on le désire, par un acide pharmaceutiquement acceptable,
qui, si on le désire, est soumis à une réaction d'hydrogénation catalytique préférentiellement à température et pression ambiantes, et en présence de Nickel de Raney en milieu d'alcool aliphatique inférieur ou de dioxanne pour obtenir un dérivé de formule (I/D) :

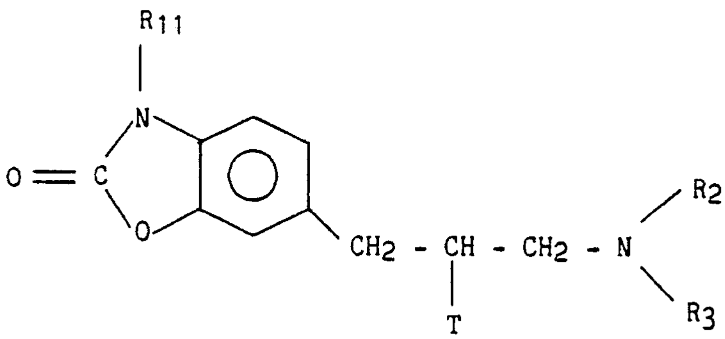

(I/D)

dans laquelle :
$R_{11}$, $R_2$, $R_3$ et T ont la même signification que précédemment,
X, Y et Z représentent chacun simultanément un atome d'hydrogène.
dont on sépare le cas échéant les isomères lorsque T ne représente pas un atome d'hydrogène,
et, que l'on salifie éventuellement par un acide pharmaceutiquement acceptable, ou que l'on transforme en sel d'ammonium quaternaire par action d'un agent d'alkylation comme indiqué précédemment.

2/ Procédé de préparation des dérivés de formule (I/D), selon la revendication 1 à partir des dérivés de formule (IX) :

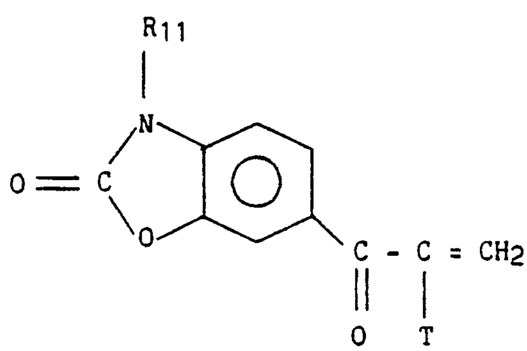

(IX)

dans laquelle :
$R_{11}$ et T ont la même signification que dans la formule (I),
que l'on traite par un hydracide pour obtenir un dérivé de formule (IV) :

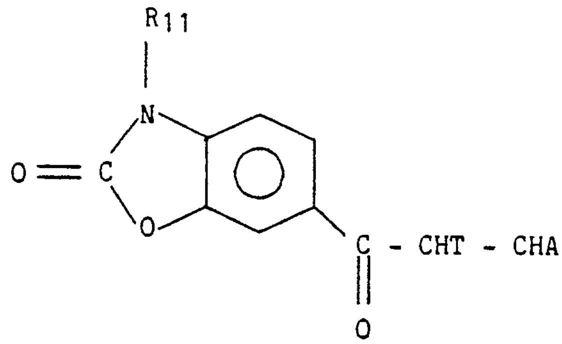

(IV)

dans laquelle $R_{11}$ et T one la même signification que précédemment et A la même signification que dans la formule (III),
dont on sépare, si on le désire, les isomères lorsque T ne représente pas un atome d'hydrogène,
que l'on soumet à réduction par un trialkylsilane en milieu acide pour conduire à un dérivé de formule (V) :

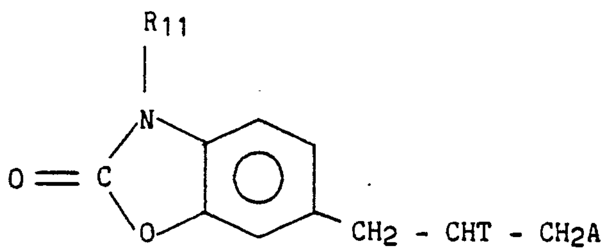

(V)

dans laquelle $R_{11}$ et T ont la même signification que précédemment et A la même signification que dans la formule (III),
que l'on soumet à l'action d'une amine de formule (VI) :

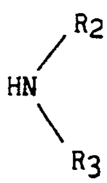

(VI)

dans laquelle $R_2$ et $R_3$ ont a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la témpérature d'ébullition du solvant choisi en présence d'un excès de l'amine choisi ou d'un capteur de l'hydracide formé,
pour conduire à un dérivé de formule (I/D) selon la revendication 1 dont on sépare les isomères si on le désire et, que l'on salifie si on le désire par un acide pharmaceutiquement acceptable ou que l'on transforme en sel d'ammonium quaternaire par action d'un agent d'alkylation.

3/ Procédé de préparation selon la revendication 1 de composés de formules (I), dans lesquels X et Y représentent simultanément un atome d'hydrogène, leurs énantiomères, diastéréoisomères et épimères, leurs sels d'ammonium quaternaire, leurs sels d'addition à un acide pharmaceutiquement acceptable.

4/ Procédé de préparation selon la revendication 1 de composés de formule (I), dans lesquels X représente un atome d'hydrogène et Y un groupement hydroxyle, leurs énantiomères, diastéréoisomères et épimères, leurs sels d'ammonium quaternaire, leurs sels d'addition à un acide pharmaceutiquement acceptable.

5/ Procédé de préparation selon la revendication 1 de composés de formule (I), dans lesquels X et Y simultanément représentent un atome d'oxygène, leurs énantiomères, diastéréoisomères et épimères, leurs

sels d'ammonium quaternaire, leurs sels d'addition à un acide pharmaceutiquement acceptable.

6/ Procédé de préparation selon la revendication 1 de composés de formule (I), dans lesquels Y forme avec Z une liaison $\pi$, leurs énantiomères, diastéréosimères et épimères, leurs sels d'ammonium quaternaire, leurs sels d'addition à un acide pharmaceutiquement acceptable.

7/ Procédé de préparation selon l'une des revendications 1 et 2 de composés de formule (I) qui est la méthyl - 3 (morpholino - 3 propyl) -6 benzoxazolinone ainsi que ses sels d'addition à un acide pharmaceutiquement acceptable.

Office européen des brevets

# RAPPORT DE RECHERCHE EUROPEENNE

Numero de la demande

EP 90 40 0535

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | ACTA THERAPEUTICA, vol. 6, no. 3, 1980, pages 205-211, Bruxelles, BE; M. CAZIN et al.: "Etude in vitro de l'effet inhibiteur de diverses beta-amino-cétones sur l'agrégation des plaquettes" * En entier * | 1,4,10, 11 | C 07 D 263/58<br>A 61 K 31/42 |
| X | EUROPEAN JOURNAL OF MEDICINAL CHEMISTRY, vol. 11, no. 1, janvier-février 1976, pages 33-42, Paris, FR; J.-C. CAZIN et al.: "Etude chimique et pharmacodynamique de beta-amino-cétones de structure benzoxazolinique" * En entier * | 1,4,10, 11 | |
| D,A | EP-A-0 110 781 (RIOM LABORATOIRES-CERM) * Revendications * | 1,10,11 | |
| D,A | EP-A-0 049 203 (CERM) * Revendications * | 1,10,11 | **DOMAINES TECHNIQUES RECHERCHES (Int. Cl.5)**<br>C 07 D 263/00 |

Le présent rapport a été établi pour toutes les revendications

| Lieu de la recherche | Date d'achèvement de la recherche | Examinateur |
|---|---|---|
| LA HAYE | 16-05-1990 | HENRY J.C. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant